# EUROPEAN PATENT SPECIFICATION

(11) **EP 2 621 957 B1**
(45) Date of publication and mention of the grant of the patent: **02.06.2021**
(21) Application number: 11833071.1
(22) Date of filing: 28.09.2011
(51) Int. Cl.: C07K 19/00, C07K 14/435, A01K 67/04, C12N 15/12, C12N 15/63

(54) **CHIMERIC SPIDER SILK AND USES THEREOF**
CHIMÄRE SPINNENSEIDE UND VERWENDUNGEN DAVON
SOIE D'ARAIGNÉE CHIMÉRIQUE ET SES UTILISATIONS

(30) Priority: 28.09.2010 US 387332 P
(43) Date of publication of application: 07.08.2013
(73) Proprietor: University of Notre Dame du Lac, South Bend, IN 46617 (US)
(72) Inventor: FRASER, Malcolm James, Granger Indiana 46530 (US); LEWIS, Randy, Laramie Wyoming 82071 (US); JARVIS, Don, Laramie Wyoming 82071 (US); THOMPSON, Kimberly, Lansing Michigan 48933 (US); HULL, Joseph, Maricopa Arizona 85138 (US); MIAO, Yun-Gen, Hangzhou Zhejiang 310029 (CN); TEULE, Florence, Laramie Wyoming 82071 (US); SOHN, Bonghee, Laramie Wyoming 82071 (US); KIM, Youngsoo, Laramie Wyoming 82071 (US)
(74) Representative: Held, Stephan
(86) International application number: PCT/US2011/053760
(87) International publication number: WO 2012/050919

(56) References cited:
- WO-A1-03/074699
- WO-A1-2008/113145
- WO-A1-2009/097540
- WO-A2-2006/008163
- JP-A- H10 179 171
- KRISTINA SPIESS ET AL: "Recombinant Spider Silk Proteins for Applications in Biomaterials", MACROMOLECULAR BIOSCIENCE, vol. 10, no. 9, 9 September 2010 (2010-09-09), pages 998-1007, XP055015288, ISSN: 1616-5187, DOI: 10.1002/mabi.201000071
- FLORENCE TEULÉ ET AL: "Modifications of spider silk sequences in an attempt to control the mechanical properties of the synthetic fibers", JOURNAL OF MATERIALS SCIENCE, KLUWER ACADEMIC PUBLISHERS, BO, vol. 42, no. 21, 17 July 2007 (2007-07-17) , pages 8974-8985, XP019528790, ISSN: 1573-4803, DOI: 10.1007/S10853-007-1642-6
- LAZARIS A ET AL: "Spider silk fibers spun from soluble recombinant silk produced in mammalian cells", SCIENCE, AMERICAN ASSOCIATION FOR THE ADVANCEMENT OF SCIENCE, vol. 295, no. 5554, 18 January 2002 (2002-01-18), pages 472-476, XP002243786, ISSN: 0036-8075, DOI: 10.1126/SCIENCE.1065780
- KRISTINA SPIESS ET AL.: 'Recombinant Spider Silk Proteins for Applications in Biomaterials' MACROMOLECULAR BIOSCIENCE vol. 10, 09 September 2010, pages 998 - 1007, XP055015288

## Description

The United States government may own rights to the technology in the present application as work was supported by grant # R21 EB007247 from the National Institute of Biomedical Imaging and Bioengineering, National Institutes of Health (DLJ). A collaborative research agreement is in place between the University of Notre Dame Office of Research (MJF), and a research agreement with Kraig BioCraft Laboratories, Inc. (MJF).

### FIELD OF THE INVENTION

The present invention relates to the field of silk fibers, as chimeric spider silk fibers with improved strength and flexibility characteristics are provided. In addition, the invention relates to the field of methods of producing chimeric silk fibers, as a method for producing an improved silk fiber (in particular, a silkworm/spider silk chimeric fiber) employing an engineered transgenic silkworm having specific spider silk genetic sequences (spider silk strength and/or spider silk flexibility and/or elasticity motif sequences), is provided. The invention also relates to transgenic organisms, as transgenic silkworms engineered to include a chimeric silkworm sequence that includes spider silk genetic sequences that are specific for spider silk flexibility and/or elasticity motifs and spider silk strength motifs, and a method for creating these transgenic silkworm employing a specifically designed piggyBac vector, are described. Commercial production methods for the chimeric silk fibers employing the transgenic silk worms described are also provided.

### BACKGROUND OF THE INVENTION

Silk fibers have been used for many years as sutures for a wide variety of important surgical procedures. Finer fibers are needed as sutures for ocular, neurological, and cosmetic surgeries. Silk fibers also hold great promise as materials for artificial ligaments, artificial tendons, elastic bandages for skin grafts in burn patients, and scaffolds that can provide support and, in some cases, temporary function during regeneration of bone, periodontal, and connective tissues. The development of silk fibers as materials for ligaments and tendons is expected to become increasingly important as the incidence of anterior cruciate ligament (ACL) and other joint injuries requiring surgical repairs increases in the ageing population. While a small proportion of fibers currently used as sutures is derived from natural silkworm silk, most are produced as synthetic polymers by the chemical industry. A major limitation of this approach is that it can only provide silk fibers with a narrow range of physical properties, such as diameter, strength, and elasticity.

A wide variety of recombinant systems, including bacteria (Lewis, et al. 1996), yeast (Fahnestock and Bedzyk, 1997), baculovirus-infected insect cells (Huemmerich, et al. 2004), mammalian cells (Lazaris, et al. 2002) ad transgenic plants (Scheller, et al. 2001) has been used to produce various silk proteins. However, none of these systems is naturally designed to spin silk and, accordingly, none has reliably produced useful silk fibers. In order for a silk fiber to be considered useful from a commercial standpoint, the fiber must posses adequate tensile (strength) and flexibility and/or elasticity characteristics, and be suitable for the creation of fibers in the desired commercial application. Thus, a need continues to exist for a system that can be used for this purpose.

Spider silk proteins have been produced in several heterologous protein production systems. In each case, the amount of protein produced is far below practical commercial levels. Transgenic plant and animal expression systems could be scaled up, but even in these systems, recombinant protein production levels would have to be increased substantially to be cost-effective. An even more difficult problem is that prior production efforts have yielded proteins, but not fibers. Thus, the proteins must be spun into fibers using a post-production method. Due to these production and spinning problems, there remains no example of a recombinant protein production system that can produce spider silk fibers long enough to be of commercial interest; i.e., "useful" fibers.

Prior reported attempts to produce fibers used a mammalian cell system to express genes encoding MaSpl, MaSp2, and related silk proteins from the spider, A. diadematus (Lazaris, et al. 2002). This work resulted in production of a 60 Kd spider silk protein, ADF-3, which was purified and used to produce fibers with a post-production spinning method. However, this system does not yield useful fibers consistently. In addition, this approach is problematic due to the need to solubilize the proteins, develop successful spinning conditions, and conduct a post-spin draw to get fibers with useful properties.

The art remains devoid of a commercial method for consistently providing silk fiber production with the requisite tensile and flexibility characteristics needed for use in manufacturing.

The WO 2009/097540 A1 discloses methods, compositions, and systems for transforming silkworms to produce spider silk and analogs of spider silk.

Wen H. et al (2010): Transgenic silkworms (Bombyx mori) produce recombinant spider dragline silk in cocoons, Molecular Biology Reports 37 (4): 1815-1821 discloses germline-transgenic silkworms (Bombyx mori) that are able to spin cocoons containing recombinant spider silk.

### SUMMARY OF THE INVENTION

The present invention overcomes the above and other difficulties described in the art. In particular, a transgenic silkworm production system adaptable to commercial magnitude is provided that circumvents the problems associated with protein purification, solubilization, and artificial post-production spinning, as it is naturally equipped to spin silk fibers.

In a general and overall sense, the present invention provides a biotechnological approach for the production of chimeric spider silk fibers using a transgenic silkworm as a platform for heterologous silk protein production of commercially useful chimeric silk fibers with superior tensile and flexibility characteristics. The chimeric silk fibers may be custom designed to provide a fiber having a specific range of desired physical properties or with pre-determined properties, optimized for the biomedical applications desired.

### Spider/Silkworm Silk Protein and Chimeric Spider Silk Fibers:

In one aspect, the invention provides a recombinant chimeric spider silk/silkworm silk protein encoded by a sequence comprising one or more spider silk flexibility and/or elasticity motif/domain sequences and/or one or more spider silk strength domain sequences. The chimeric spider/silkworm silk protein is described as encoding a Spider 6 chimeric spider/silkworm silk protein. Further disclosed are a Spider 2, Spider 4 or Spider 8 chimeric spider/silkworm silk protein.

In addition, the present invention provides for chimeric spider silk fibers prepared from the chimeric silk worm/spider silk proteins. In particular embodiments, the chimeric spider silk fibers are described as having greater tensile strength as compared to native silkworm silk fibers, and in some embodiments, up to 2-fold greater tensile strength as compared to native silkworm fibers.

### Transgenic Silk Worms:

The transgenic organism is a transgenic silk worm, which is a transgenic *Bombyx mori.* The host silkworm that is to be transformed to provide the transgenic silkworm may be a mutant silkworm that lacks the ability to produce native silk fibers. The silkworm mutant may pnd-wl.

The mutant silkworm *(B. mori)* will be transformed using a *piggyBac* system, wherein a *piggyBac* vector is prepared using an expression cassette that contains a synthetic spider silk protein sequence flanked by N- and C-terminal fragments of the *B. mori* fhc protein. Generally, the silkworm transformation involves introducing a mixture of the *piggyBac* vector and a helper plasmid, encoding the *piggyBac* transposase, into pre-blastoderm embryos by microinjecting silkworm eggs. An eppendorf robotic needle manipulator calibrated to puncture the Chorion is used to create a micro-insertion opening through which a glass capillary is inserted through which a DNA solution is injected into the silkworm egg. The injected eggs are then allowed to mature, and Progress to hatch into larvae. The larvae are permitted to mature to mature silk worms, and spin cocoons according to routine life cycle of the silk worm.

Cross-breeding of these transgenic insects with each other, or with non-transgenic insects/silk worms, are also provided as part of the present disclosure.

### Spider Silk Genetic Expression Cassettes:

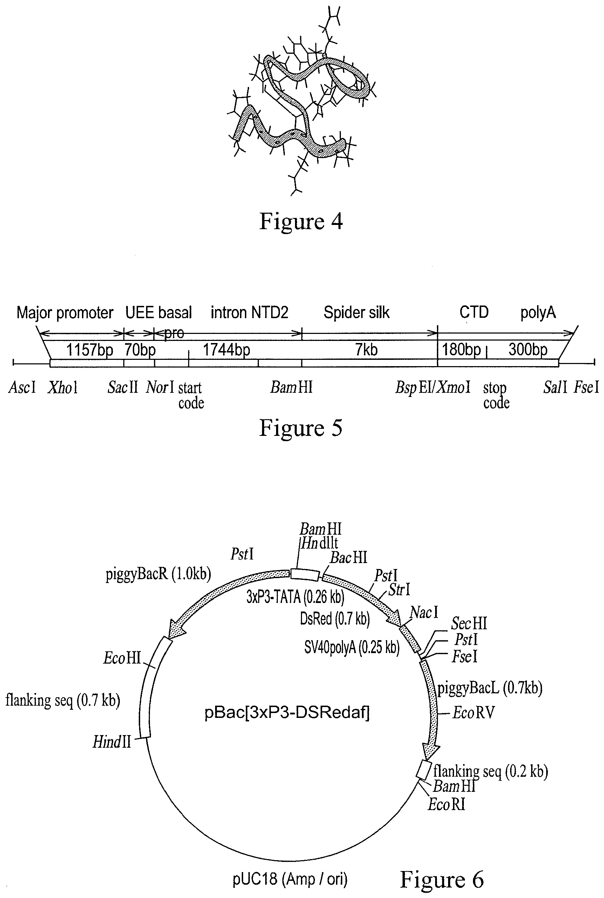

In another aspect, chimeric silk worm/spider silk expression cassettes are provided, the cassette comprising one or more spider silk protein sequence motifs that correspond to one or more of a number of particular spider silk flexibility and/or elasticity motif sequences and/or spider silk strength motif sequences as disclosed herein. In another aspect, methods for producing a chimeric spider silk/silkworm protein and fiber are provided. At least eight (8) different versions of the expression cassette as depicted in Figure 5 have been provided, which encode four different synthetic spider silk proteins with or without EGFP inserted in-frame between the NTD abd spider silk sequences. These sequences are identified herein as "Spider 2 (reference example)", "Spider 4(reference example)", "Spider 6" and Spider 8(reference example)".

### Transgenic Silk Worms:

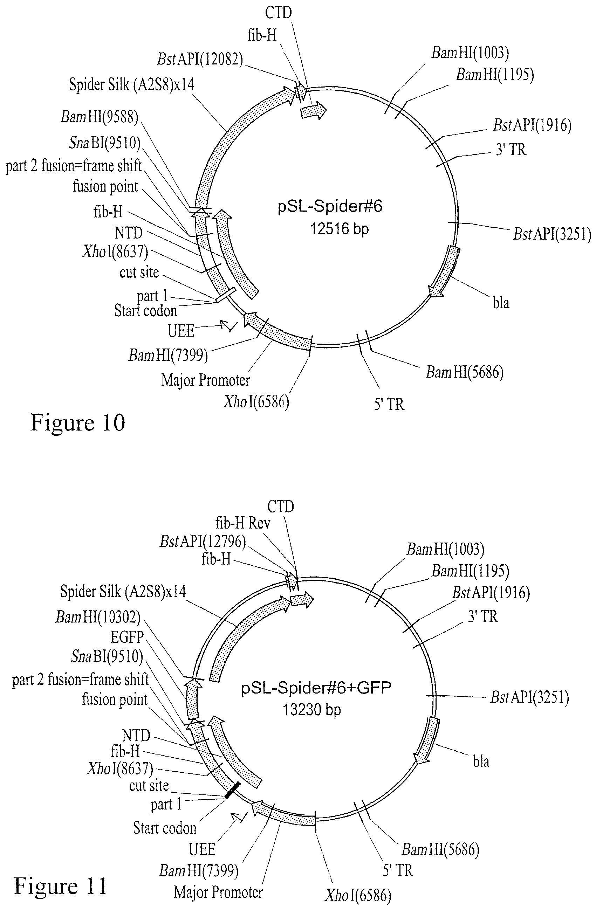

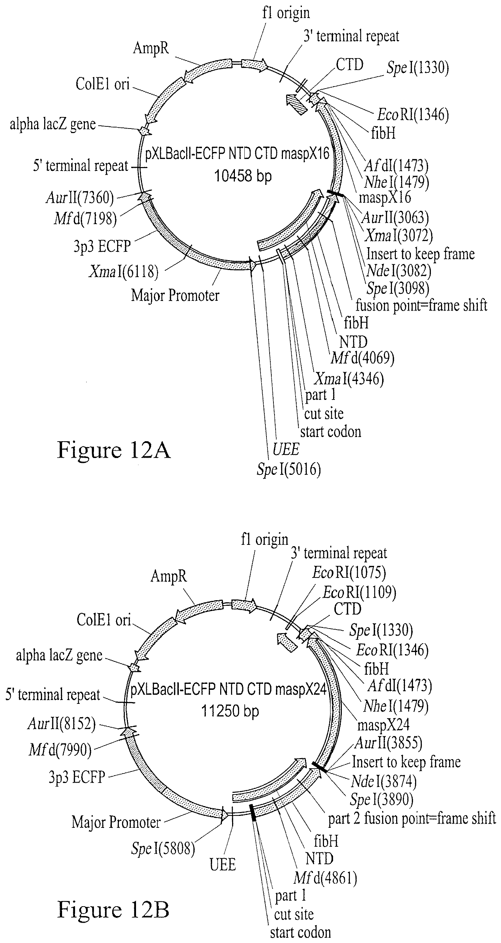

In yet another aspect, a transgenic silkworm and methods for preparing a transgenic silkworm are provided. The method of preparing a transgenic silkworm comprises: preparing an expression cassette having a sequence comprising a silkworm sequence, a chimeric spider silk sequence encoding one or more spider silk strength motif sequences and one or more spider silk flexibility and/or elasticity motif sequences, subcloing said cassette sequence into a *piggyBac* vector (such as a *piggyBac* vector pBac[3xP3-DsRedaf], see Figure 6, see Figures 10-11 for parent plasmids, See Figures 12A-12B for plasmids subcloned from parent plasmids, introducing a mixture of *the piggyBac* vector and a helper plasmid encoding *apiggyBac* transposase, into a pre-blastoderm silkworm embryo (e.g., by microinjecting silkworm eggs), maintaining the injected silkworm embryo under normal rearing conditions (about 28° C and 70% humidity) until larvae hatch, and obtaining a transgenic silk worm.

These transgenic silk worms may be further mated to generate Fl generation embryos for subsequent identification of putative transformants, based an expression of the S-Red eye marker. Putative male and female transformants identified by this method are then mated to produce homozygous lineages for more detailed genetic analysis. Specifically, silkworm transformation involved injecting a mixture of the *piggyBac* vector and helper plasmid DNA's into silkworm eggs of a clear cuticle silkworm mutant, pnd-wl. The silkworm mutant, pnd-wl, was described in Tamura, et al. 2000, this reference being specifically incorporated herein in its entirety. This mutant has a melanization deficiency that makes screening using fluorescent genes much easier. Once red-eyed, putative F 1 transformants were identified, homozygous lineages were confirmed using Western blotting of silk gland proteins and harvested cocoon silk.

### Methods of Manufacturing Chimeric Spider Silk /Silkworm Silk Fibers:

In yet another aspect, the invention provides a commercial production method for producing chimeric spider silk/ silkworm fibers in a transgenic silk worm. In one embodiment, the method comprises preparing the transgenic silk worms described herein, and cultivating the transgenic silk worms under conditions that permit them to grow and form cocoons, harvesting the cocoons, and obtaining the chimeric spider silk fibers from the cocoons. Standard techniques for unraveling and/or otherwise harvesting silk fibers from a silk cocoon may be used.

### Articles of Manufacture and Methods of Using Same:

Further, a variety of articles of manufacture are disclosed made from the chimeric spider silk fibers as described before. For example, the recombinant chimeric spider/silkworm fibers may be used in medical suture materials, wound dressings and tissue/joint replacement and reconstructive materials and devices, drug delivery patches and/or other delivery item, protective clothing (bullet-proof vests and other articles), recreational articles (tents, parachutes, camping gear, etc.), among other items.

Further disclosed are methods of using the recombinant chimeric spider silk/silkworm fibers in various medical procedures. For example, the fibers may be used to facilitate tissue repair, in growth or regeneration as scaffold in a
tissue engineered biocompatible construct prepared with the recombinant fibers, or to provide delivery of a protein or therapeutic agent that has been engineered into the fiber.

Unless otherwise defined, all technical and scientific terms used herein have the same meaning as commonly understood by one of ordinary skill in the art. Although methods and materials similar or equivalent to those described herein can be used in the practice or testing of the invention, the preferred methods and materials are described below. In addition, the materials, methods and examples are illustrative only and not intended to be limiting. In case of conflict, the present specification, including definitions, controls.

### BRIEF DESCRIPTION OF THE DRAWINGS

Other objects and advantages of the present invention will become apparent to those skilled in the art upon reading the following detailed description of preferred embodiments, in conjunction with the accompanying drawings, wherein like reference numerals have been used to designate like elements, and wherein:

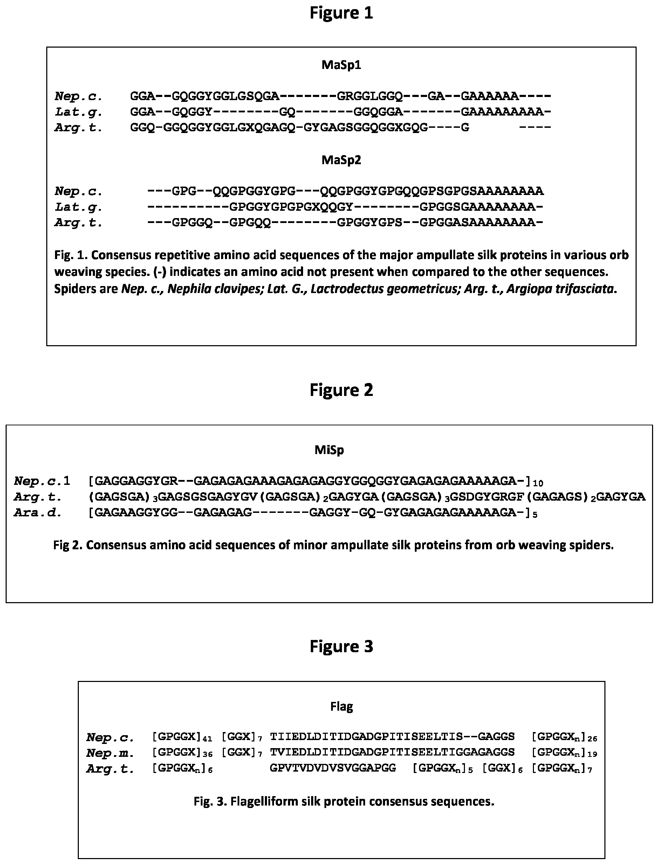

FIG. 1 presents the amino acid sequences of the two major ampullate silk proteins from divergent orb weaving or derived orb weaving spiders (Gatesy, et al. 2001). Comparison reveals a high level of sequence conservation, particularly within the sequence motifs described above, which has been maintained over the 125 million years since these species diverged from one another. Consensus repetitive amino acid sequences of the major ampullate silk proteins in various orb weaving species (-) indicates an amino acid not present when compared to the other sequences. Spiders are: *Nep. c, Nephila ciavipes; Lat.g., Lactrodectus geornetricus; Arg. t., Argiope trifasciata.*
FIG. 2 - presents consensus amino acid sequences of minor ampullate silk proteins from orb weaving spiders. Soon after the initial major ampullate silk protein sequences were published, cDNAs representing minor ampullate silk (Mi) protein transcripts from *N. ciavipes* were isolated and sequenced (Colgin and Lewis, 1998). The MiSp sequence provided in this figure has both similar and conspicuously different sequences relative to the MaSp proteins. MiSp includes GGX and short polyAla sequences, but the longer polyAla motifs in the MaSps are replaced by (GA)n repeats. The consensus repeats have similar organizations but the number of GGX
   and GA repeats varies greatly.
FIG. 3 - presents flagelliform silk protein cDNA consensus sequences. These silk protein cDNAs encode the catching spiral silk protein from the *N. clavipes* flagelliform gland (Fig. 3; Hayashi and Lewis, 2000). These cDNAs contained sequences encoding a 5' untranslated region and a secretory signal peptide, numerous iterations of a five amino acid motif, and the C-terminal end. Northern blotting analysis indicated an mRNA size of -15 kb, encoding a protein of nearly 500 Kd. The amino acid sequence predicted from the gene sequence suggested a model of protein structure that helps to explain the physical basis for the elasticity of spider silk, which also is consistent with the properties of MaSp2 (further described herein).
FIG. 4 - presents a computer model of a **β** spiral. This is a model of an energy minimized (GPGGQGPGGY)2 sequence, with a starting configuration of Type II B-turns at each pentamer sequence.
FIG. 5 - presents several variations on a basic *Bombyx mori* silk fibroin heavy chain expression cassette that were constructed. The design involved the assembly of constructs designed to express fibroin heavy chain (fhc)-spider silk chimeras, in which the synthetic spider silk protein sequence is flanked by N- and C-terminal fragments of the B.mori fhc protein. The functionally relevant genetic elements in each expression cassette, from left to right, include: the major promoter, upstream enhancer element (UEE), basal promoter, and N-terminal domain (NTD) from the *B. mori* fhc gene, followed by various synthetic spider silk protein sequences positioned in-frame with the translational initiation site located upstream in the NTD, followed by the fhc C-terminal domain (CTD), which includes translational termination and RNA polyadenylation sites.
FIG. 6 - presents the scheme for subcloning the cassettes *into piggyBac.* Each of the eight different versions of the expression cassette pictured were excised from a parent plasmid using AscI and Fsel and subcloned into the corresponding sites of pBAC[3xP3-DSRedaf]. A map of *thispiggyBac* vector is shown.

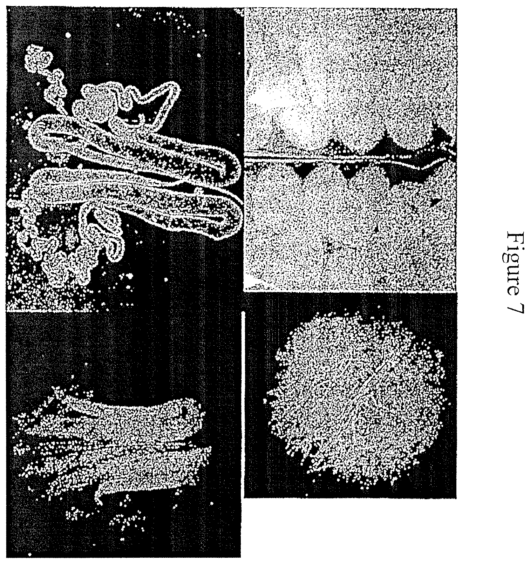

FIG. 7 - presents a Western blot of transgenic silkworm silks. These silks were analyzed for the presence of the spider silk chimeric protein by western blotting of both the silkworm silk gland protein contents and the silk fibers from transgenic silkworm cocoons using a spider silk-specific antibody. In both cases, transgenic silkworms were verified as producing the chimeric proteins, and differential extraction studies showed that these proteins were integral components of the transgenic silk fibers of their cocoons. Furthermore, expression of each of the chimeric green fluorescent protein fusions was apparent in both silk glands and fibers by direct examination of the silk glands or silk fibers using a fluorescent dissecting microscope. In most cases the amount of fluorescent protein in the fibers was high enough to be visualized by the green color the coccons under normal lighting.

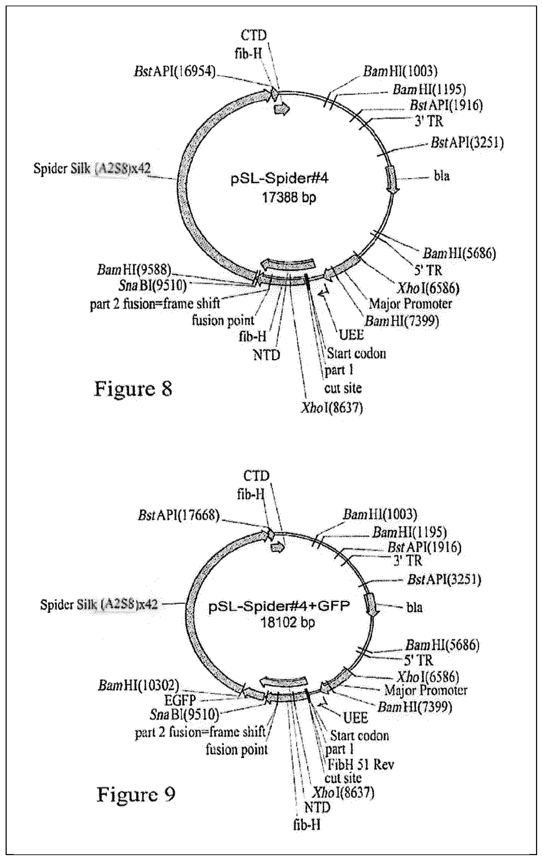

FIG. 8 - presents a parent plasmid pSL-Spider #4, a size of 17,388 bp. This parent plasmid carries the chimeric spider silk protein #4 cassette, Spider silk (A2S8)x42.
Figure 9 - presents a parent plasmid pSL-Spider#4 + GFP. GFP is Green Fluorescent Protein. This vector has a size of 18,102 bp. This parent plasmid carries the chimeric spider silk protein #4 with the marker protein, GFP, cassette, Spider silk (A2S8)x42.
Figure 10 - presents a parent plasmid pSL-Spider#6. This parent plasmid has a size of 12,516 bp. This parent plasmid carries the chimeric spider silk protein #6 cassette, Spider silk (A2S8)x14.
Figure 11 - presents a parent plasmid pSL-Spider#6 + GFP. GFP is Green Fluorescent Protein. This parent plasmid has a size of 13,230 bp. This parent plasmid carries the chimeric spider silk protein #6 with the marker protein, GFP, cassette, Spider silk (A2S8)x14.
Figure 12A - B - presents *the piggyBac* plasmids. Figure 12A depicts the pXLBacII-ECFP NTD CTD maspX16 construct having a size of 10,458 bp. Figure 12B depicts the pXLBacII-ECFP NTD CTD maspX24 construct, and has a size of 11,250 bp.
Figure 13 - presents the sequence for pSL-Spider#4.
Figure 14 - presents the sequence for pSL-Spider#4+GFP
Figure 15 - presents the sequence for pSL-Spider#6.
Figure 16 - presents the sequence for pSL-Spider#6+GFP.

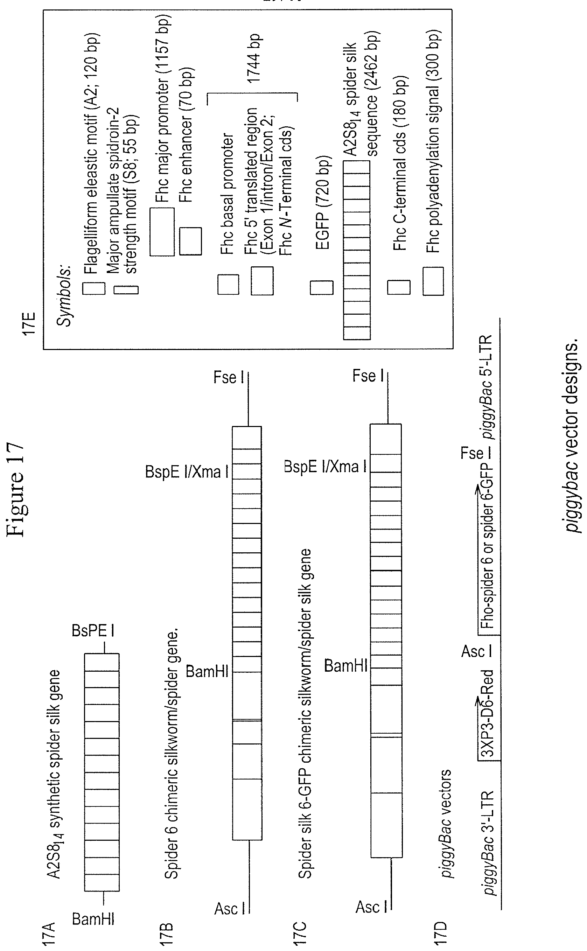

Figure 17 - presents *the piggyBac* vector designs. Figure 17A A2S8₁₄ synthetic spider silk gene; Figure 17B. Spider 6 chimeric silkwomilspider silk gene; Figure 17C. Spider silk 6-GFP chimeric silkwomilspider silk gene; Figure 17D. *piggyBac* vectors; Figure 17E Symbols for: Flagellum elastic motif (A2; 120 bp); Major ampullate spidroin-2; Spider motif (S8; 55 bp) Fhc major promoter (1,157 bp), Fhc enhancer (70 bp); Fhc basal promoter, Fhc 5' translated region (Exon 1/intron/Exon 2; Fhc N-terminal cds) = 1,744 bp; EGFP (720 bp); A2S8₁₄. spider silk sequence (2,462 bp), Fhc C-terminal cds (180 bp), Fhc polyadenylation signal (300 bp).
Figure 18 - presents expression of the chimeric silkworm/spider silk/EGFP protein in (18A) cocoons, (18B, 18C) silk glands, and (18D) silk fibers from spider 6-GFP silkworms. Expression and localization of a chimeric silkworm/spider silk protein in silkworm silk glands. Silk glands were excised, bombarded with the spider 6 or spider *6-GFPpiggyBac* vectors, and examined under a fluorescence microscope, as described in Methods.

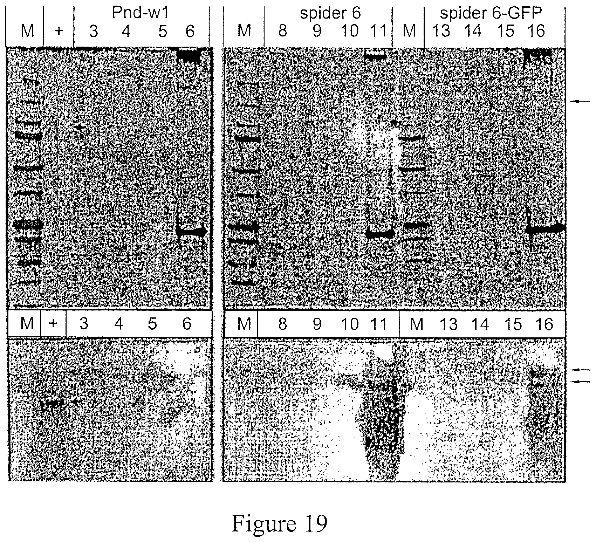

Figure 19 - Sequential extraction of silk fibers. Cocoons produced by pnd-w 1 (lanes 3-6), spider 6 (lanes 8-1 1), or spider 6-GFP (lanes 13-16) silkworms were degummed and subjected to a sequential extraction protocol, as described herein. Proteins solubilized in each extraction step were analyzed by SDSPAGE and (19A) Coomassie Blue staining or (19B) immunoblotting with a spider silk protein-specific antiserum. M: Molecular weight markers. +: A2S8 14 spider silk protein expressed and purified in *E. coli.* Lanes 3, 8, and 13: sahne extractions. Lanes 4, 9, and 14: SDS extractions. Lanes 5, 10, and 15: 8M LiSCN/2% mercaptoethanol extractions. Lanes 6, 11, and 16: 16M LiSCN/5% mercaptoethanol extractions. The arrows mark the chimeric spider silk proteins. The apparent molecular weights were -75 kDa for A2S814 from *E. coli,* -106 kDa for spider 6, and -130 kDa and -110 kDa for spider 6-GFP.

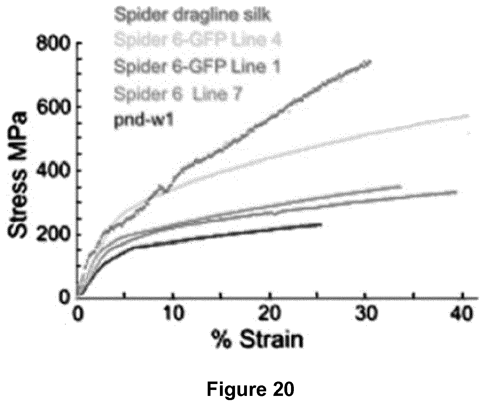

Figure 20 - A comparison of the best mechanical performances observed for the composite fibers from the transgenic silkworms, the native fibers from the parental silkworm, and a representative native (dragline) spider silk fiber is shown. Fiber toughness is defined by the area under the stress/strain curves. Mechanical properties of degummed native and composite silk fibers. The best mechanical performances measured for the native silkworm (pnd-wl) and representative spider (*N*. *clavipes* dragline) silk fibers are compared to those obtained with the composite silk fibers produced by transgenic silkworms. All fibers were tested under the same conditions. The toughest values are: spider 6 line 7 (86.3 MJ/rm); spider 6-GFP line 1 (98.2 MJ/m3), spider 6-GFP line 4 (167.2 MJ/m3); and *N. clavipes* dragline (138.7 MJ/rm), as compared to native silkworm pnd-wl (43.9 MJ/rm). These data show that all of the composite silk fibers from transgenic silkworms were tougher than the native fibers from the non-transgenic silkworm.

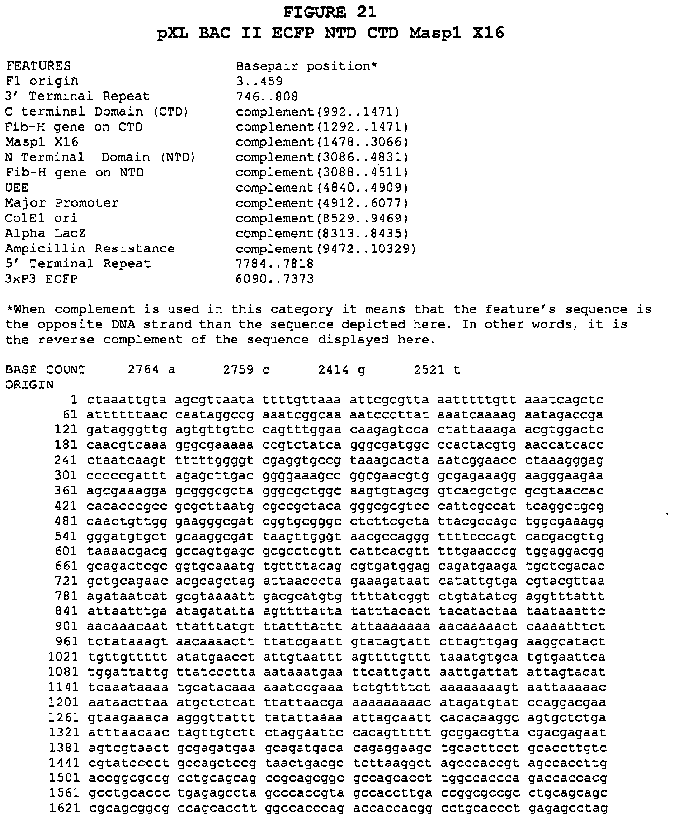

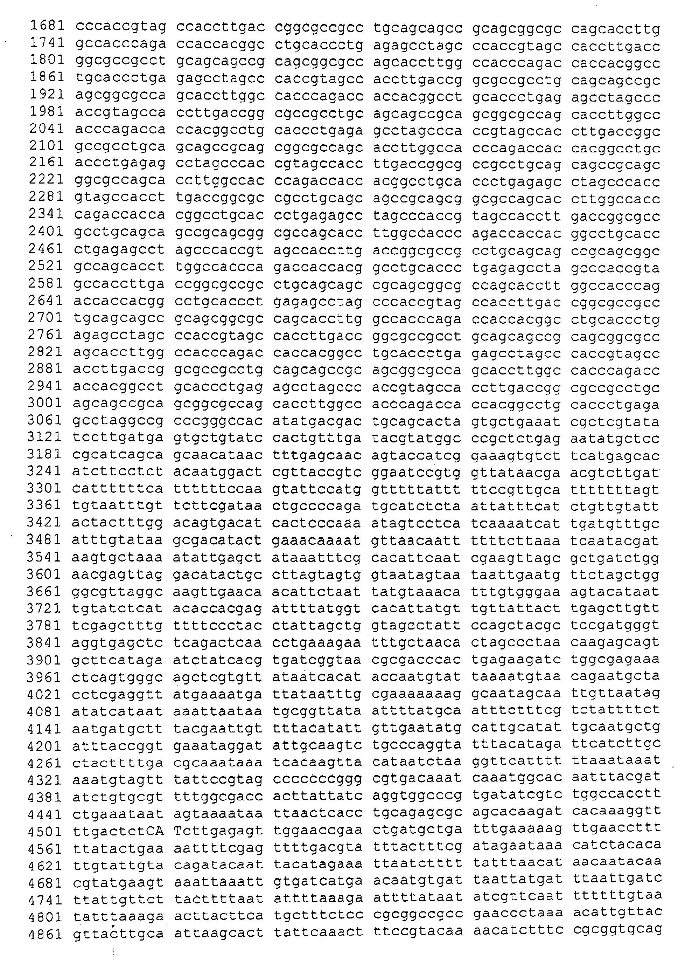

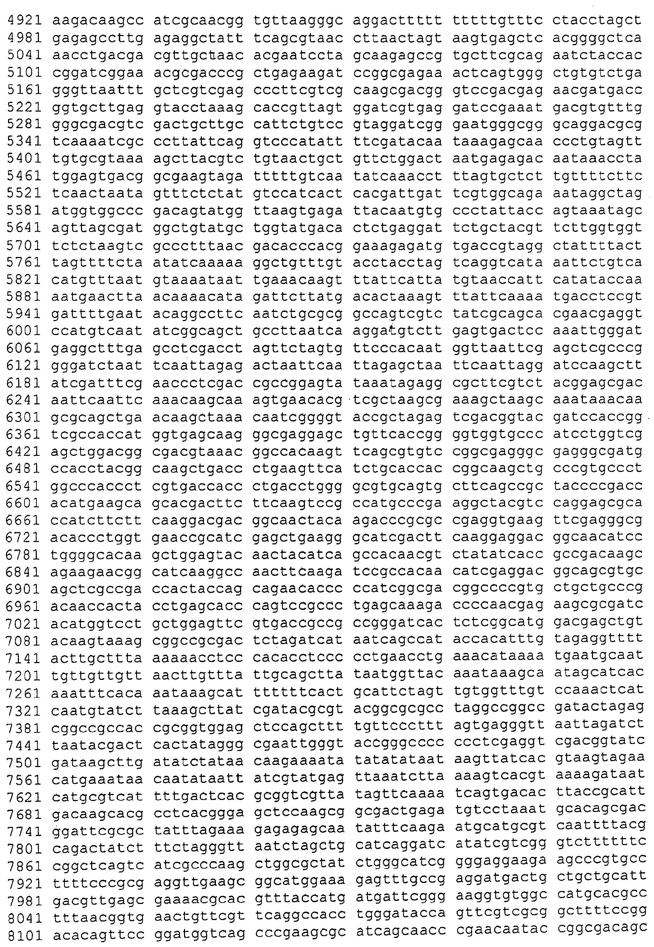

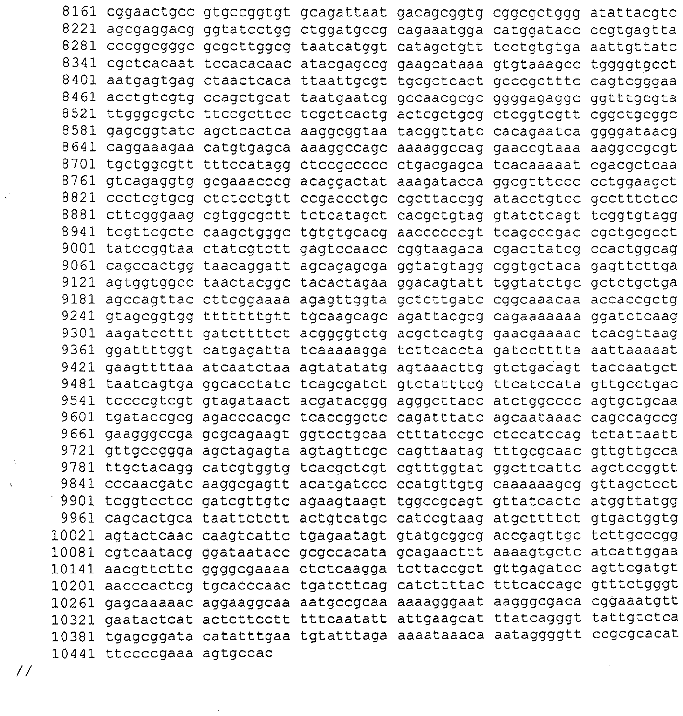

Figure 21- depicts the nucleic acid sequence of construct pXLBacII-ECFP NTD CTD maspX16 (10,458 pb).

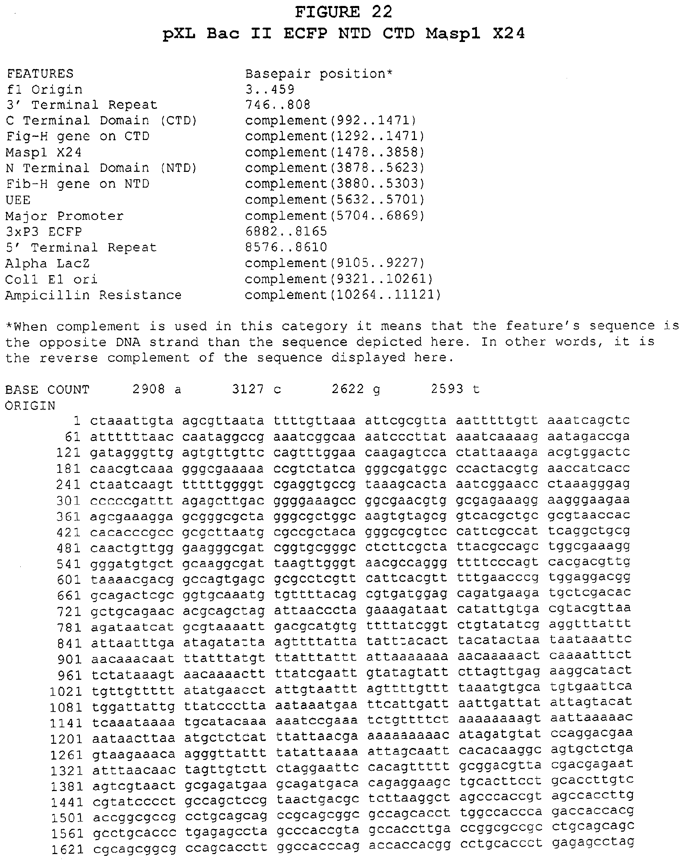

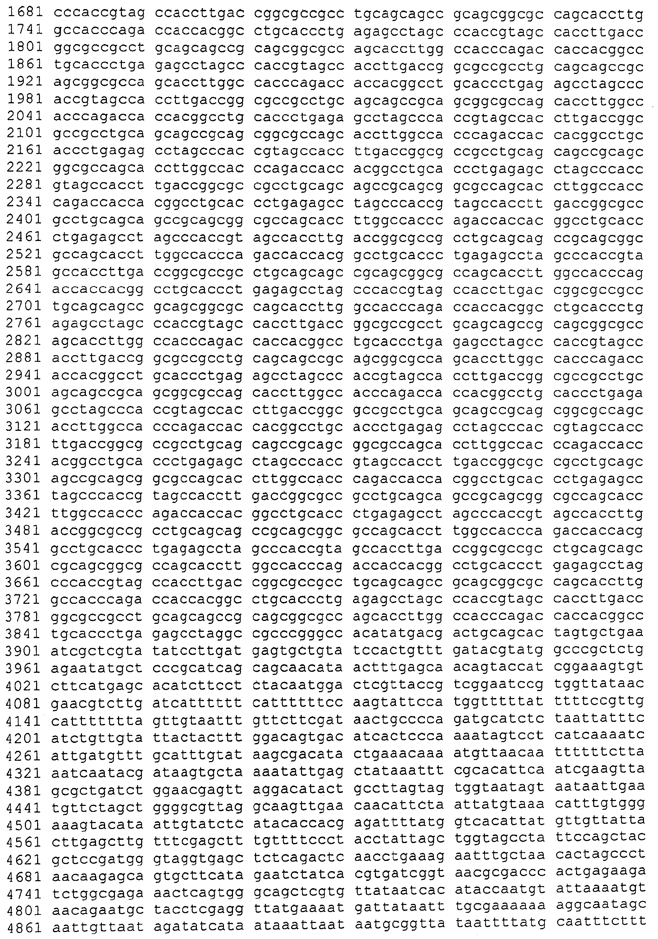

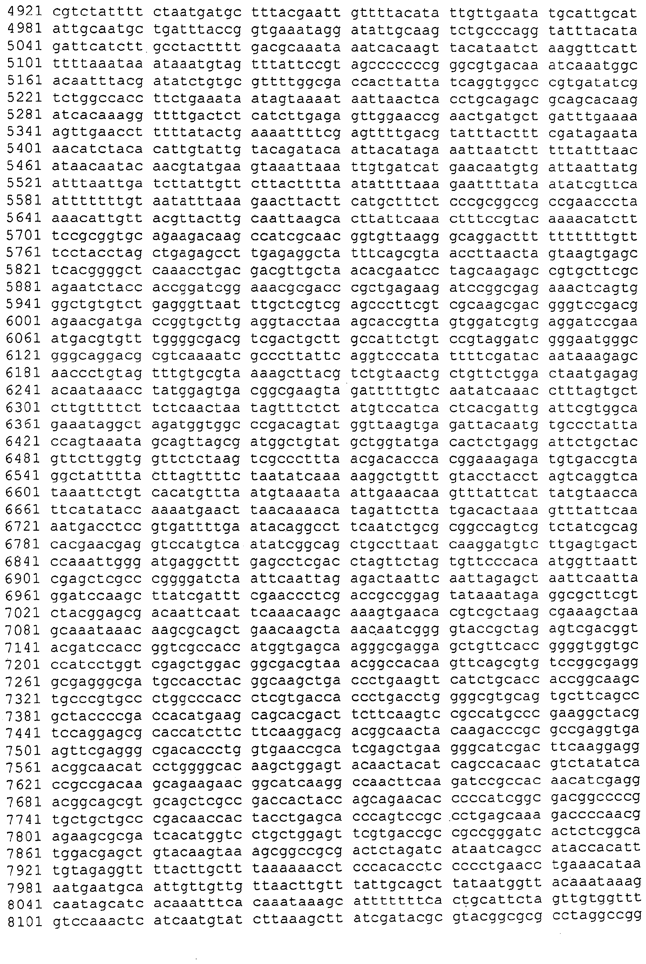

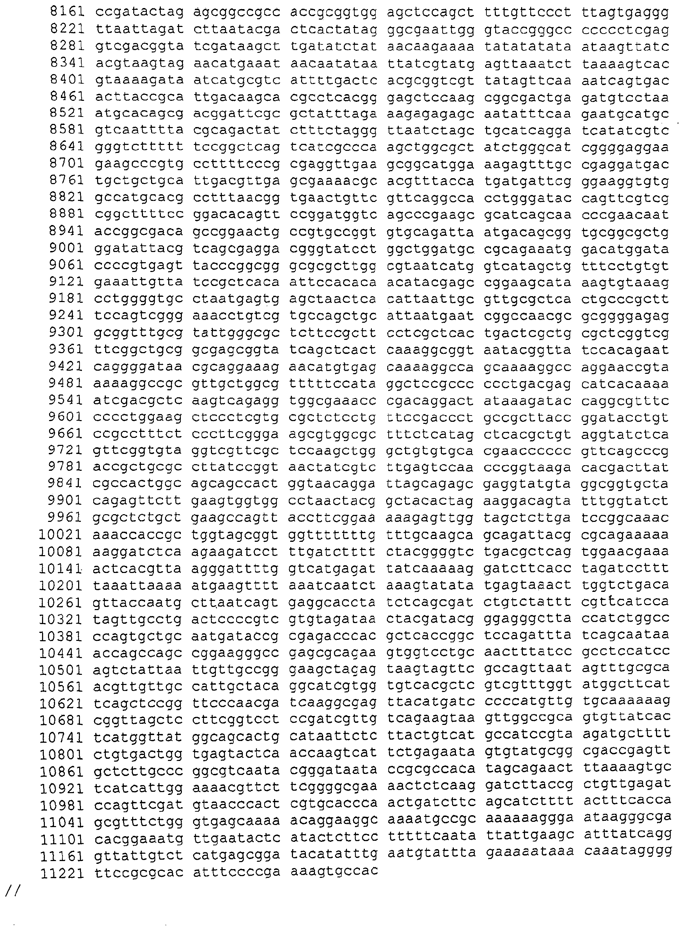

Figure 22 - depicts the nucleic acid sequence of construct pXLBacII-ECFP NTD CTD maspX24 (11,250 bp).

### DETAILED DESCRIPTION OF THE PREFERRED EMBODIMENTS

The method for inserting a gene into silkworm chromosomes used in the present invention should enable the gene to be stably incorporated and expressed in the chromosomes, and be stably propagated to offspring, as well, by mating. Although a method using micro-injection into silkworm eggs or a method using a gene gun can be used, a method that is used preferably consists of the micro-injection into silkworm eggs with a target gene containing vector for insertion of an exogenous gene into silkworm chromosomes and helper plasmid containing a transposon gene (Nature Biotechnology 18, 81-84, 2000) simultaneously.

The target gene is inserted into reproductive cells in a recombinant silkworm that has been hatched and grown from the micro-injected silkworm eggs. Offspring of a recombinant silkworm obtained in this manner are able to stably retain the target gene in their chromosomes. The gene in the recombinant silkworm obtained in the present invention can be maintained in the same manner as ordinary silkworms. Namely, up to fifth instar silkworms can be raised by incubating the eggs under normal conditions, collecting the hatched larva to artificial feed and then raising them under the same conditions as ordinary silkworms.

The recombinant silkworm obtained in the present invention can be raised in the same manner as ordinary silkworms, and is able to produce exogenous protein by raising under ordinary conditions, to maximize silkworm development and growth.

Gene recombinant silkworms obtained in the present invention are able to pupate and produce a cocoon in the same manner as ordinary silkworms. Males and females are distinguished in the pupa stage, and alter having transformed into moths, males and females mate and eggs are gathered on the following day. The eggs can be stored in the same manner as ordinary silkworm eggs. The gene recombinant silkworms of the present invention can be maintained on subsequent generations by repeating the breeding as described above, and can be increased to large numbers.

Although there are no particular limitations on the promoter used here, and any promoter originating in any organism can be used provided its acts effectively
within silkworm cells, a promoter that has been designed to specifically induce protein in silkworm silk glands is preferable. The silkworm silk gland protein promoters ist fibroin H chain promoter. Further disclosed are fibroin L chain promoter, p25 promoter and sericin promoter.

In the present invention, a "gene cassette for expressing a chimeric spider silk protein" refers to a set of DNA required for a synthesis of the chimeric protein in the case of being inserted into insect cells. This gene cassette for expressing an a chimeric spider silk protein contains a promoter that promotes expression of the gene encodes the chimeric spider silk protein. Normally, it also contains a terminator and poly A addition region, and preferably contains a promoter, exogenous protein structural gene, terminator and poly A addition region. Moreover, it may also contain a secretion signal gene coupled between the promoter and the exogenous protein structural gene. An arbitrary gene sequence may also be coupled between the poly A addition sequence and the exogenous protein structural gene. In addition, an artificially designed and synthesized gene sequence can also be coupled.

In addition, a "gene cassette for inserting a chimeric spider silk/silkworm gene" refers to a gene cassette for expressing a chimeric spider silk/silkworm gene having an inverted repetitive sequence of a pair of *piggyBac* transposons on both sides, and consisting of a set of DNA inserted into insect cell chromosomes through the action of the *piggyBac* transposons.

A vector in the present invention refers to that having a cyclic or linear DNA structure. A vector capable of replicating in E. Coli and having a cyclic DNA structure is particularly preferable. This vector can also incorporate a marker gene such as an antibiotic resistance gene or jellyfish green fluorescence protein gene for the purpose of facilitating selection of transformants.

Although there are no particular limitations on the insect cells used in the present invention, they are Bombyx mori cells, and more preferably silkworm silk gland cells or cells contained in Bombyx mori eggs. Further disclosed are lepidopteron cells. In the case of silk gland cells, posterior silk gland cells of fifth instar silkworm larva are preferable because there is active synthesis of fibroin protein and they are easily handled.

There are no particular limitations on the method used to incorporate a gene cassette for expression of a chimeric spider silk protein by the insect cells. Methods using a gene gun and methods using micro-injection can be used for incorporation into cultured insect cells, in the case of incorporating into silkworm silk gland cells, for example, a gene can be easily incorporated into posterior silk gland tissue removed from the body of a fifth instar silkworm larvae using a gene gun.

Gene incorporation into the posterior silk gland using a gene gun can be carried out by, for example, bombarding gold particles coated with a vector containing a gene cassette for expressing exogenous protein into a posterior silk gland immobilized on an agar plate and so forth using a particle gun (Bio-Rad, Model No. PDS-1000/He) at an He gas pressure of 1,100 to 1,800 psi.

In the case of incorporating a gene into cells contained in eggs of Bombyx mori, a method using micro-injection is preferable. Here, in the case of performing micro-injection into eggs, it is not necessary to micro-inject into the cells of the eggs directly, but rather a gene can be incorporated by simply micro-injecting into the eggs.

A recombinant silkworm containing the "gene cassette for expressing a chimeric spider silk protein" of the present invention in its chromosomes can be acquired by micro-injecting a vector having a "cassette for inserting a chimeric spider silk gene" into the eggs of Bombyx mori. For example, a first generation (G1) silkworm is obtained by simultaneously micro-injecting a vector having a "gene cassette for inserting a chimeric spider silk gene" and a plasmid in which *apiggyBac* transposase gene is arranged under the control of silkworm actin promoter into Bombyx mori eggs according to the method of Tamara, et al. (Nature Biotechnology 18, 81-84, 2000), followed by breeding the hatched larva and crossing the resulting adult insects (GO) within the same group. Recombinant silkworms normally appear at a frequency of 1 to 2% among this GI generation.

Selection of recombinant silkworms can be carried by PCR using primers designed based on the exogenous protein gene sequence after isolating DNA from the GI generation silkworm tissue. Alternatively, recombinant silkworms can be easily selected by inserting a gene encoding green fluorescence protein coupled downstream from a promoter capable of being expressed in silkworm cells into a "gene cassette for inserting a gene" in advance, and then selecting those individuals that emit green fluorescence under ultraviolet light among GI generation silkworms at first instar stage.

In addition, in the case of the micro-injection of a vector having a "gene cassette for inserting a gene" into Bombyx mori eggs for the purpose of acquiring recombinant silkworms containing a "gene cassette for expressing an exogenous protein" in their chromosomes, recombinant silkworms can be acquired in the same manner as described above by simultaneously micro-injecting a *piggyBαc* transposase protein.

A *piggyBac* transposon refers to a transfer factor of DNA having an inverted sequences of 13 base pairs on both ends and an ORF inside of about 2.1 k base pairs. Although there are no particular limitations on the *piggyBac* transposon used in the present invention, examples of those that can be used include those originating in Trichoplusia ni cell line TN-368, Autographa californica NPV (AcNPV) and Galleria mellonea NPV (GmMNPV). *ApiggyBac* transposon having gene and DNA transfer activity can be preferably prepared using plasmids pHA3PIG and pPIGA3GFP having a portion of a *piggyBac* originating in Trichoplusia ni cell line TN-368 (Nature Biotechnology 18, 81-84, 2000). The structure of the DNA sequence originating in a *piggyBac* is required to have a pair of inverted terminal sequences containing a TTAA sequence, and has an exogenous gene such as a cytokine gene inserted between those DNA sequences. It is more preferable to use a transposase in order to insert an exogenous gene into silkworm chromosomes using a DNA sequence originating in a transposon. For example, the frequency at which a gene is inserted into silkworm chromosomes can be improved considerably by simultaneously inserting DNA capable of expressing *apiggyBac* transposase to enable the transposase transcribed and translated in the silkworm cells to recognize the two pairs of inverted terminal sequences, cut out the gene fragment between them, and transfer it to silkworm chromosomes.

The invention may be even more fully appreciated by the description that follows.

### Chimeric Silk Proteins in the Biomedical Arena:

Chimeric spider silk fibers are provided as part of a widely used material for a subset of procedures, such as ocular surgeries, nerve repairs, and plastic surgeries, which require extremely thin fibers. Additional uses include scaffolding materials for regeneration of bone, ligaments and tendons as well as materials for drug delivery.

The recombinant spider silk fibers produced by the processes of the present invention may be used in a variety of medical applications such as wound closure systems, including vascular wound repair devices, hemostatic dressings, patches and glues, sutures, drug delivery and in tissue engineering applications, such as, for example, scaffolding, ligament prosthetic devices and in products for Jong-term or bio-degradable implantation into the human body. A preferred tissue engineered scaffold is a non- woven network of the fibers prepared with the recombinant spider silk/silkworm fibers described herein.

Additionally, the recombinat chimeric silk fibers of the present invention can be used for organ repair, replacement or regeneration strategies that may benefit from these unique scaffolds, including but are not limited to, spine disc, cranial tissue, dura, nerve tissue, liver, pancreas, kidney, bladder, spleen, cardiac muscle, skeletal muscle, tendons, ligaments and breast tissues.

Further disclosed are recombinant spider silk fiber materials that can contain therapeutic agents. To form these materials, the therapeutic agent may be engineered into the fiber prior to forming the material or loaded into the material after it is formed. The variety of different therapeutic agents that can be used in conjunction with the recombinant chimeric silk fibers of the present invention is vast. In general, therapeutic agents which may be administered via the pharmaceutical compositions of the invention include, without limitation: anti-infectives such as antibiotics and antiviral agents; chemotherapeutic agents (i.e., anticancer agents); anti-rejection agents; analgesics and analgesic combinations; anti-inflammatory agents; hormones such as steroids; growth factors (bone morphogenic proteins (i.e., BMP's 1-7), bone morphogenic-like proteins (i.e., GFD-5, GFD-7 and GFD-8), epidermal growth factor (EGF), fibroblast growth factor (i.e., FGF 1-9), platelet derived growth factor (PDGF), insulin like growth factor (IGF-I and IGF-II), transforming growth factors (i.e., TGF-.beta.I-III), vascular endothelial growth factor (VEGF)); and other naturally derived or genetically engineered proteins, polysaccharides, glycoproteins, or lipoproteins. These growth factors are described in The Cellular and Molecular Basis of Bone Formation and Repair by Vicki Rosen and R. Scott Thies, published by R. G. Landes Company hereby incorporated herein by reference.

The recombinant spider silk/silkworm fibers containing bioactive materials may be formulated by mixing one or more therapeutic agents with the fiber used to make the material. Alternatively, a therapeutic agent could be coated on to the fiber preferably with a pharmaceutically acceptable carrier. Any pharmaceutical carrier can be used that does not dissolve the fiber. The therapeutic agents, may be present as a liquid, a finely divided solid, or any other appropriate physical form.

The amount of therapeutic agent will depend on the particular drug being employed and medical condition being treated. Typically, the amount of drug represents about 0.001 percent to about 70 percent, more typically about 0.001 percent to about 50 percent, most typically about 0.001 percent to about 20 percent by weight of the material. Upon contact with body fluids or tissue, for example, the drug will be released.

The tissue engineering scaffolds made with the recombinant spider silk/silkworm fibers can be further modified after fabrication. For example, the scaffolds can be coated with bioactive substances that function as receptors or chemoattractors for a desired population of cells. The coating can be applied through absorption or chemical bonding.

**Suitable** additives suitable include biologically or pharmaceutically active compounds. Examples of biologically active compounds include cell attachment mediators, such as the peptide containing variations of the "RGD" integrin binding sequence known to affect cellular attachment, biologically active ligands, and substances that enhance or exclude particular varieties of cellular or tissue ingrowth. Such substances include, for example, osteoinductive substances, such as bone morphogenic proteins (BMP), epidermal growth factor (EGF), fibroblast growth factor (FGF), platelet- derived growth factor (PDGF), vascular endothelial growth factor (VEGF), insulin-like growth factor (IGF-I and II), TGF-, YIGSR peptides, glycosaminoglycans (GAGs), hyaluronic acid (HA), integrins, selectins and cadherins.

The scaffolds are shaped into articles for tissue engineering and tissue guided regeneration applications, including reconstructive surgery. The structure of the scaffold allows generous cellular ingrowth, eliminating the need for cellular preseeding. The scaffolds may also be molded to form external scaffolding for the support of in vitro culturing of cells for the creation of external support organs.

The scaffold functions to mimic the extracellular matrices (ECM) of the body. The scaffold serves as both a physical support and an adhesive substrate for isolated cells during in vitro culture and subsequent implantation. As the transplanted cell populations grow and the cells function normally, they begin to secrete their own ECM support.

In the reconstruction of structural tissues like cartilage and bone, tissue shape is integral to function, requiring the molding of the scaffold into articles of varying thickness and shape. Any crevices, apertures or refinements desired in the three-dimensional structure can be created by removing portions of the matrix with scissors, a scalpel, a laser beam or any other cutting instrument. Scaffold applications include the regeneration of tissues such as nervous, musculoskeletal, cartilaginous, tendenous, hepatic, pancreatic, ocular, integumenary, arteriovenous, urinary or any other tissue forming solid or hollow organs.

The scaffold may also be used in transplantation as a matrix for dissociated cells, e.g., chondrocytes or hepatocytes, to create a three-dimensional tissue or organ. Any type of cell can be added to the scaffold for culturing and possible implantation, including cells of the muscular and skeletal systems, such as chondrocytes, fibroblasts, muscle cells and osteocytes, parenchymal cells such as hepatocytes, pancreatic cells (including Islet cells), cells of intestinal origin, and other cells such as nerve cells, bone marrow cells, skin cells, pluripotent cells and stem cells, and combination thereof, either as obtained from donors, from established cell culture lines, or even before or after genetic engineering. Pieces of tissue can also be used, which may provide a number of different cell types in the same structure.

The cells are obtained from a suitable donor, or the patient into which they are to be implanted, dissociated using standard techniques and seeded onto and into the scaffold. In vitro culturing optionally may be performed prior to implantation. Alternatively, the scaffold is implanted, allowed to vascularize, then cells are injected into the scaffold. Methods and reagents for culturing cells *in vitro* and implantation of a tissue scaffold are known to those skilled in the art.

The recombinant spider silk/silkworm fibers of the present intention may be sterilized using conventional sterilization process such as radiation based sterilization (i.e., gamma-ray), chemical based sterilization (ethylene oxide) or other appropriate procedures. Preferably the sterilization process will be with ethylene oxide at a temperature between 52-55 °C . for a time of 8 hours or less. After sterilization the biomaterials may be packaged in an appropriate sterilize moisture resistant package for shipment and use in hospitals and other health care facilities.

The chimeric silk fibers of the resent invention may also be sued in the manufacture of various forms of athletic and protection garments, such as in the manufacture/fabrication of athletic clothing and bulletproof vests. The chimeric spider silk fibers disclosed herein may also be used in the automobile industry, such as in improved airbag fabrication. Airbags employing the disclosed chimeric silk fibers provide greater impact energy in a car crash, much as a spider web absorbs the energy of flying insects that fall prey to the web.

### Definitions

As used herein, biocompatible means that the silk fiber or material prepared there from is nontoxic, non-mutagenic, and elicits a minimal to moderate inflammatory reaction. Preferred biocompatible polymer for use in the present invention may include, for example, polyethylene oxide (PEO), polyethylene glycol (PEG), collagen, fibronectin, keratin, polyaspartic acid, polylysine, alginate, chitosan, chitin, hyaluronic acid, pectin, polycaprolactone, polylactic acid, polyglycolic acid, polyhydroxyalkanoates, dextrans, and polyanhydrides. In accordance with the present invention, two or more biocompatible polymers can be added to the aqueous solution.

As used herein, a flexibility and/or elasticity motif and/or domain sequence is defined as an identifiable genetic sequence of a gene or protein fragment that encodes a spider silk that is associated with imparting a characteristic of elasticity and/ or flexibility to a material, such as to a silk fiber. By way of example, a flexibility and/or elasticity motifs and/or domain is GPGGA.

As used herein, a strength motif is defined as an identified genetic sequence of a gene or protein fragment encoding spider silk that is associated with imparting a characteristic of strength to a material, such as to increase and/or enhance the tensile strength to a silk fiber. By way of example, some of these spider strength motifs are: GGPSGPGS(A) 8 (when A is a poly Adenine sequence).

The invention will be further characterized by the following examples which are intended to be exemplary of the invention.

### Example 1- Materials and Methods

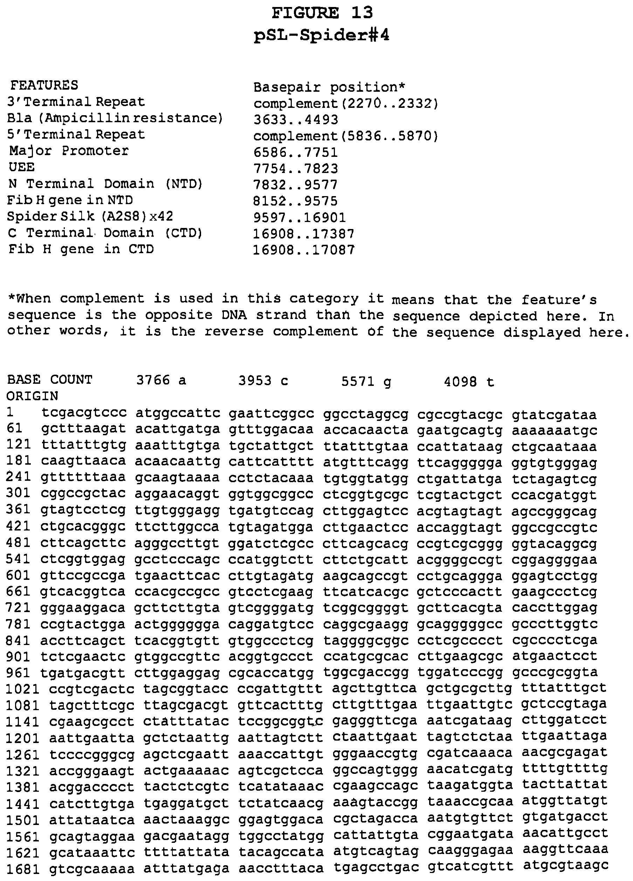

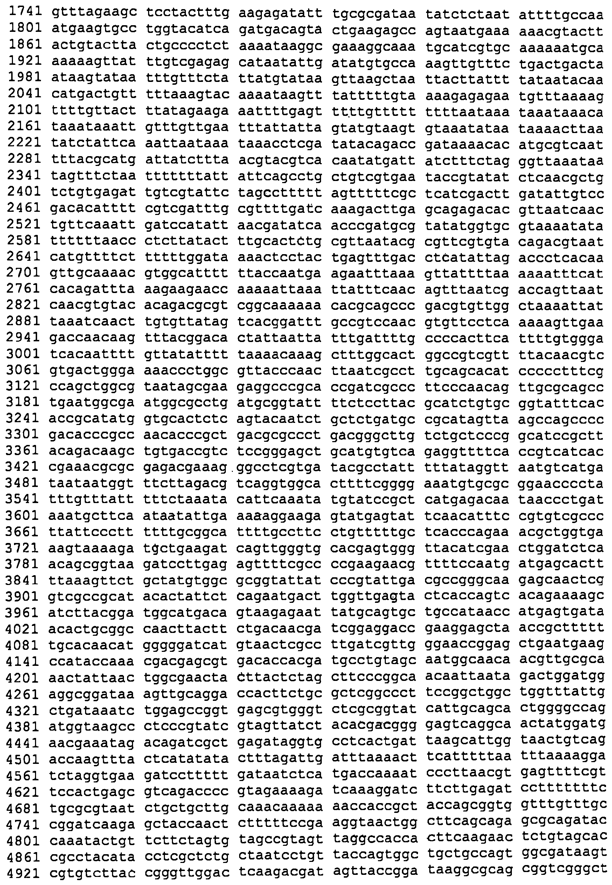

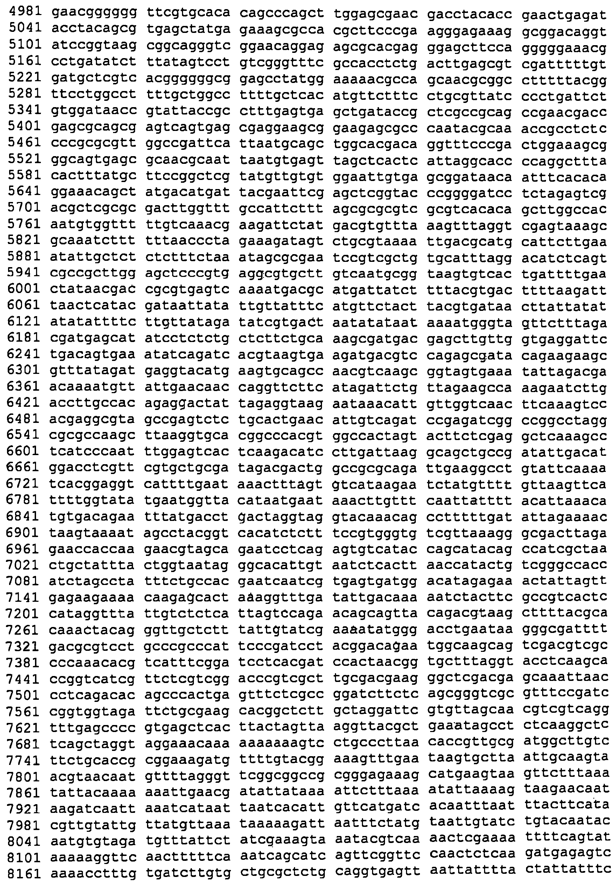

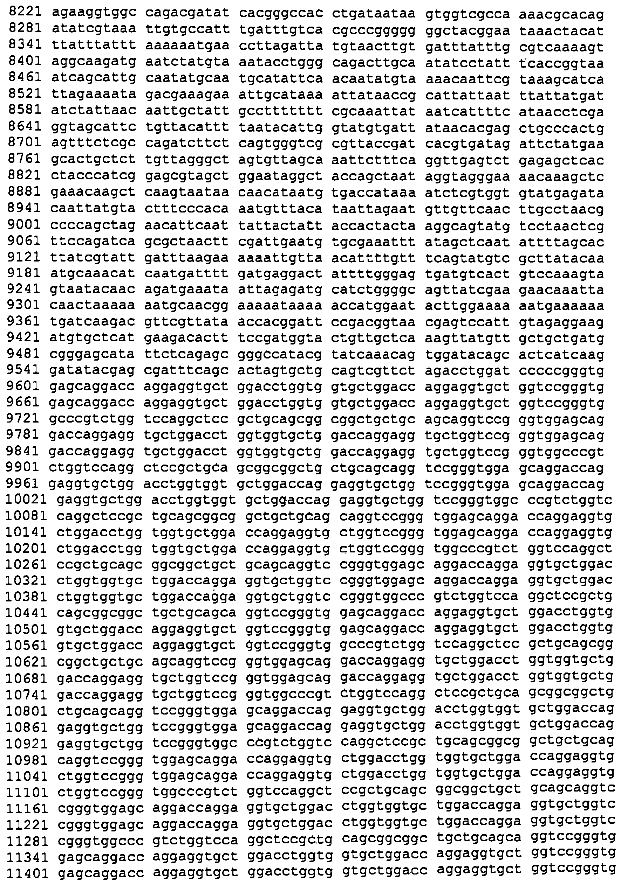

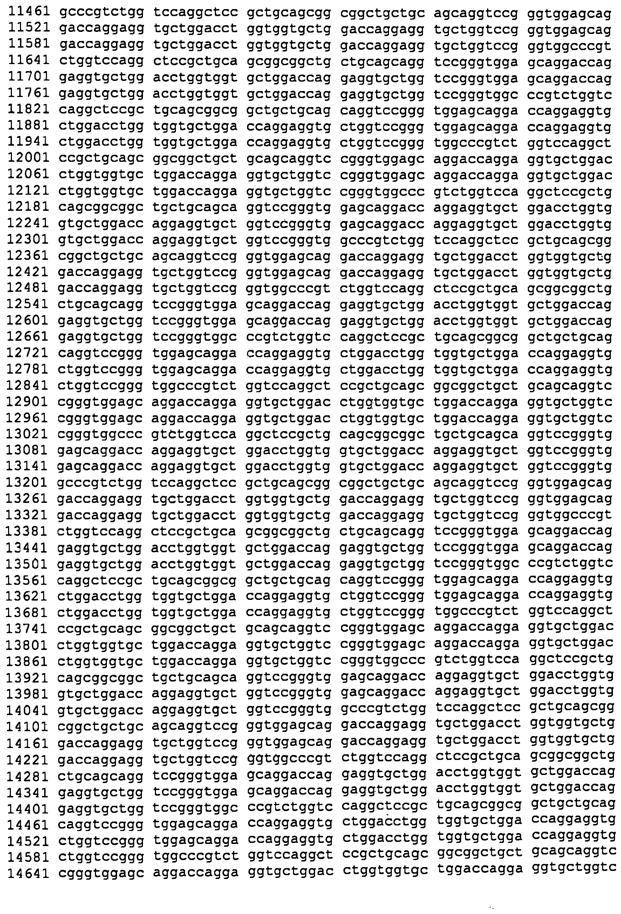

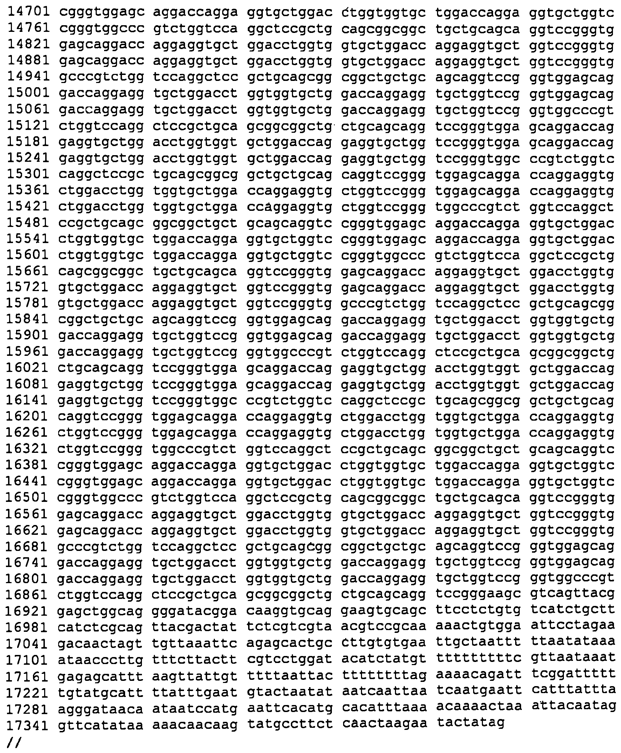

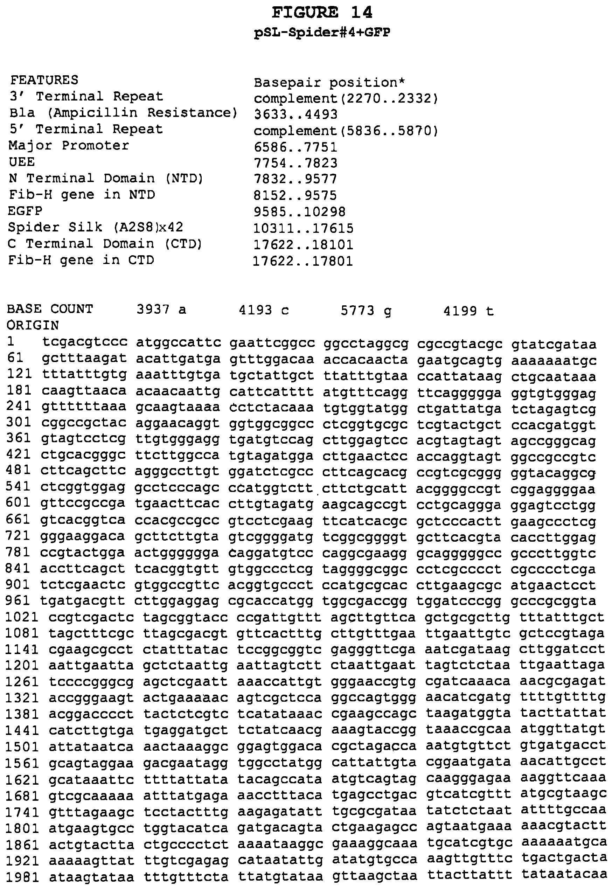

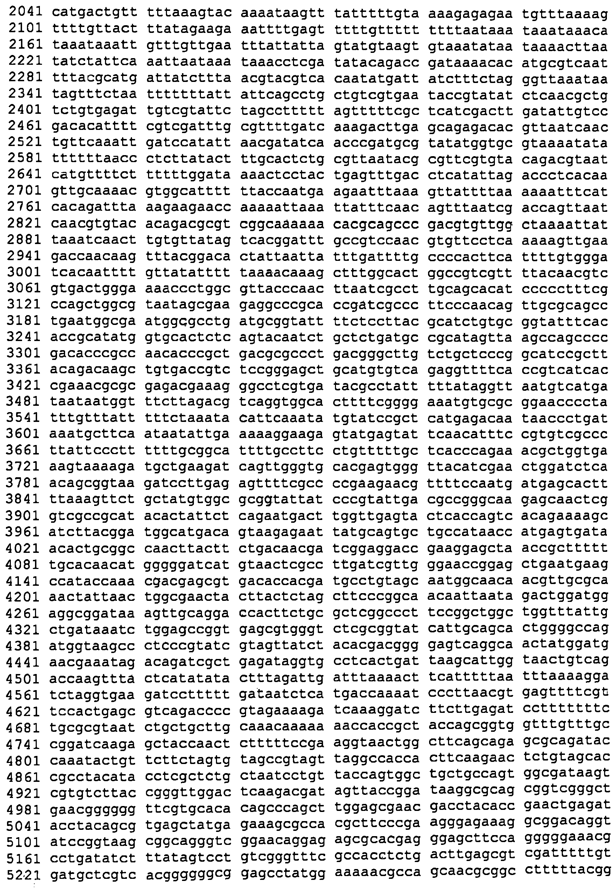

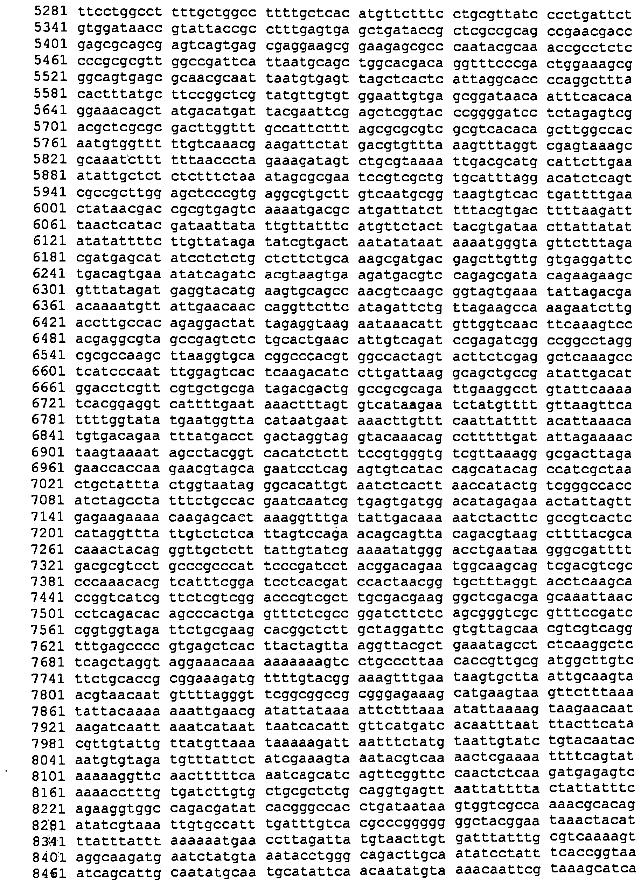

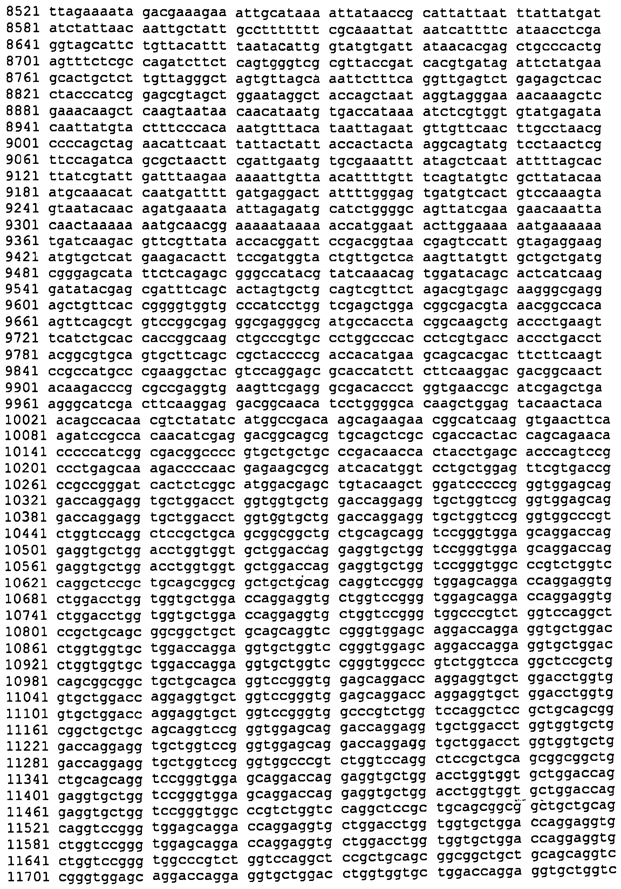

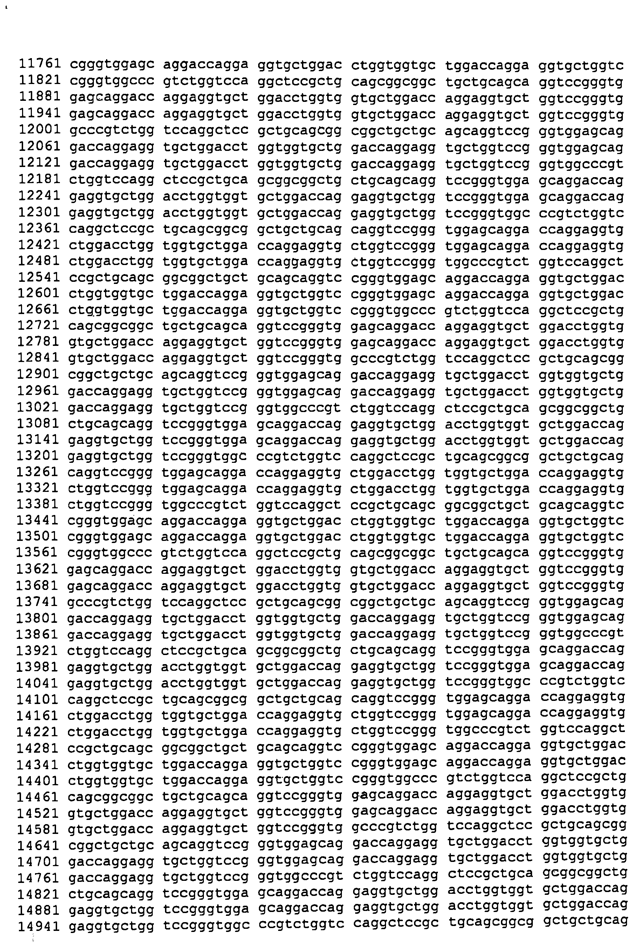

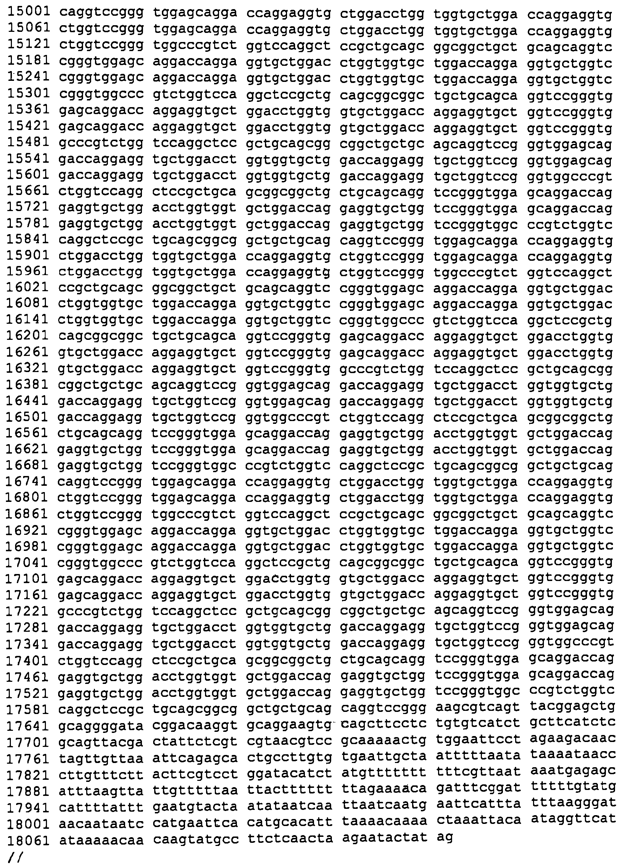

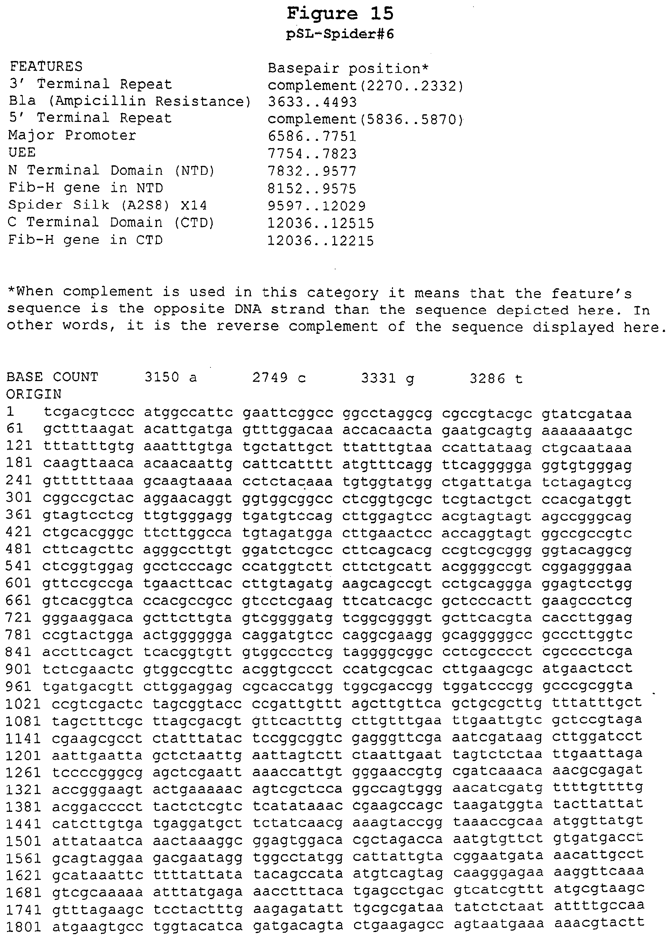

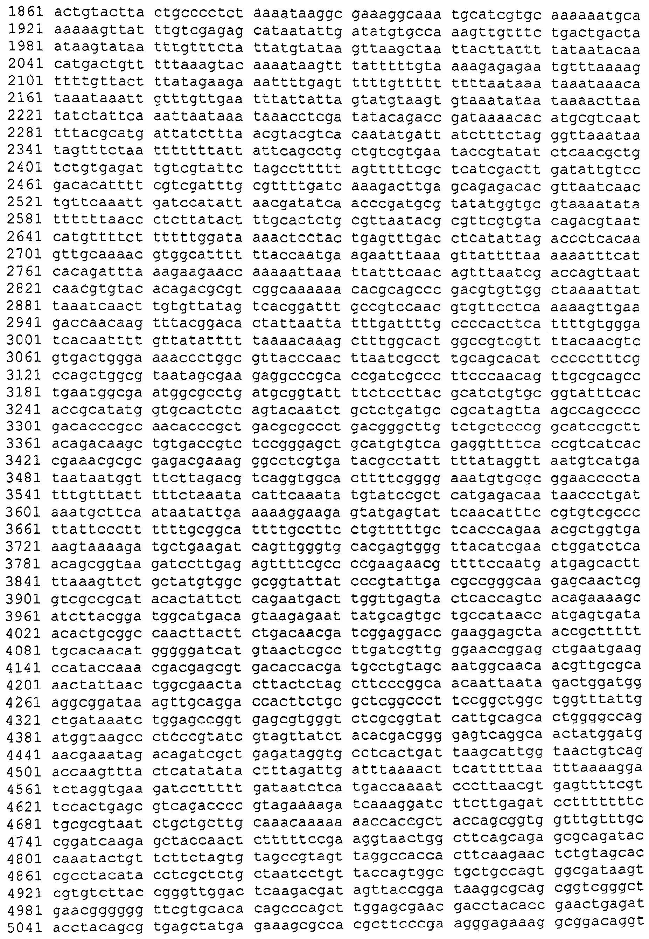

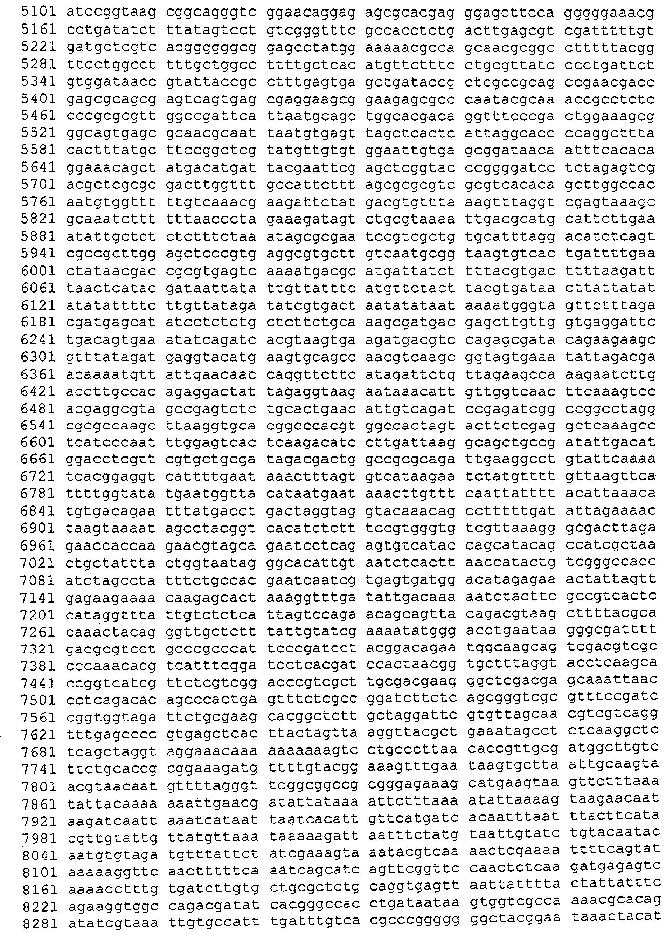

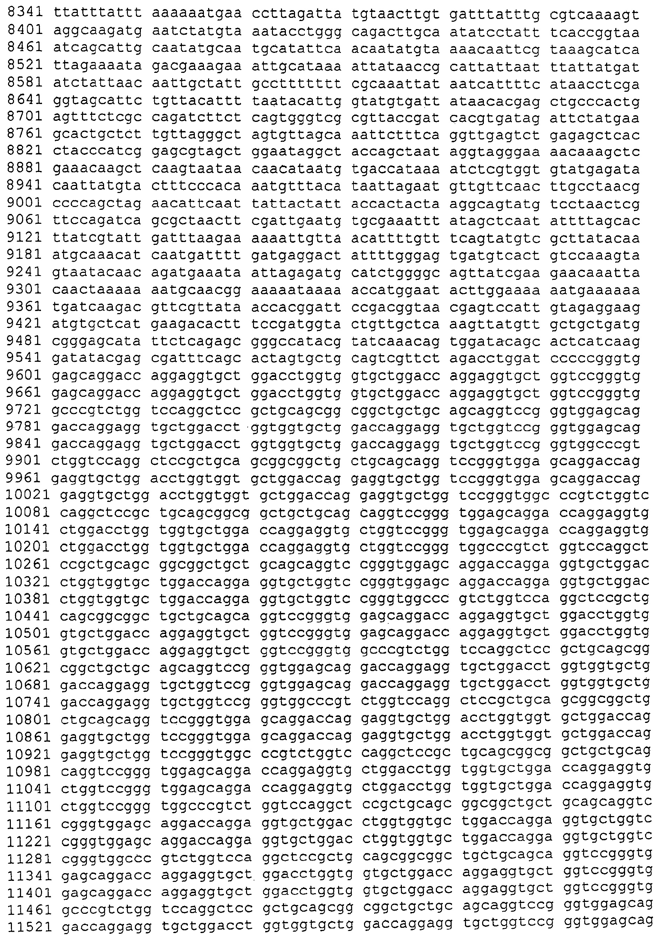

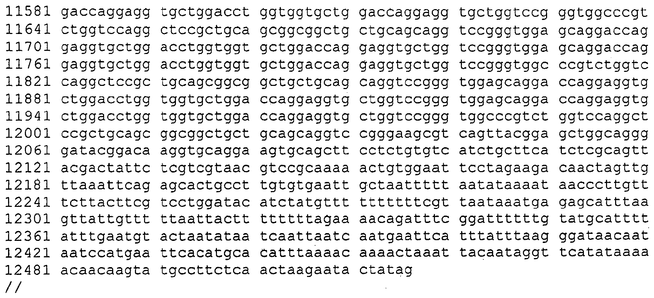

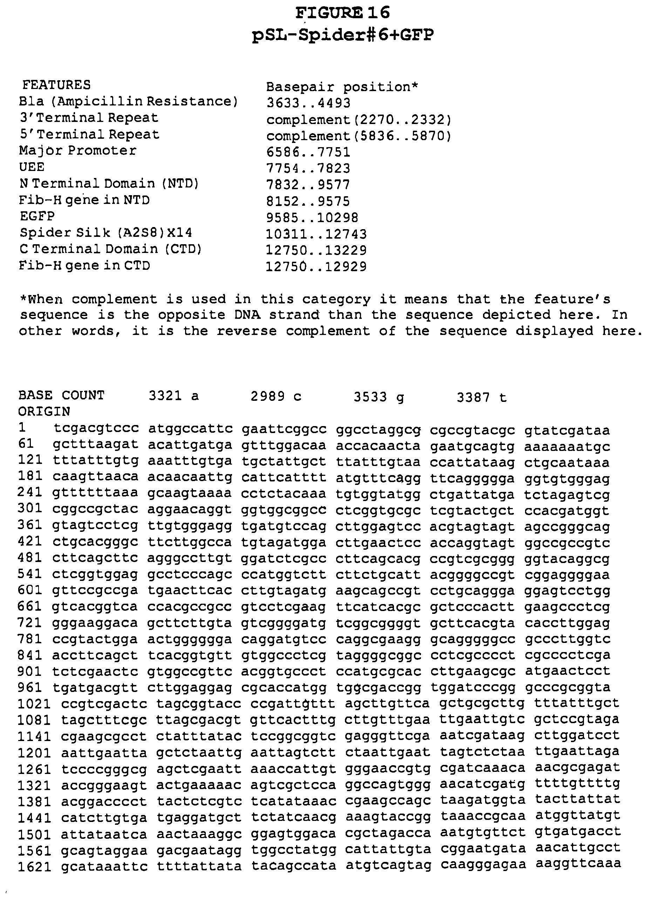

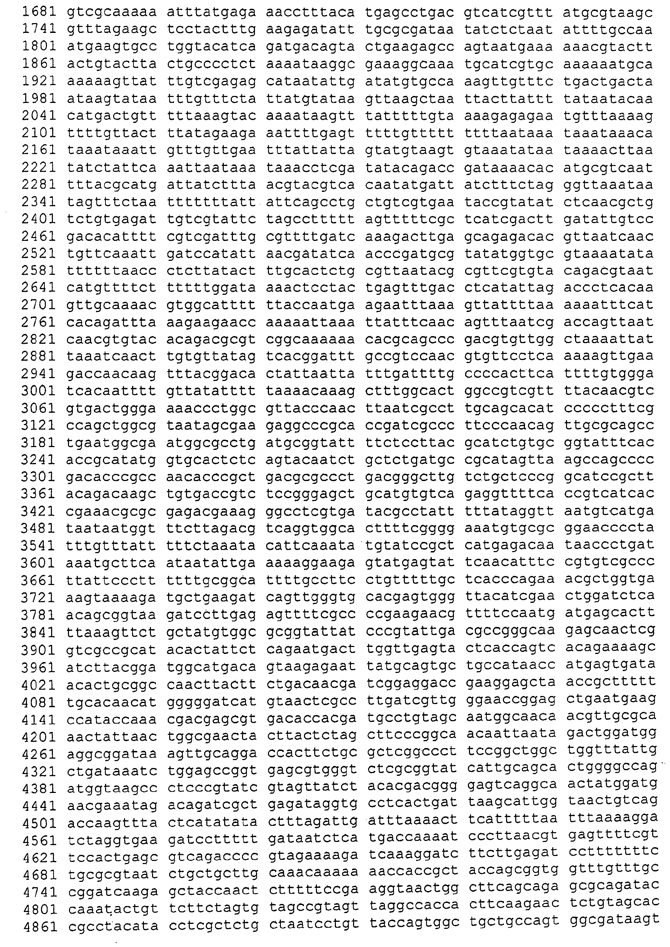

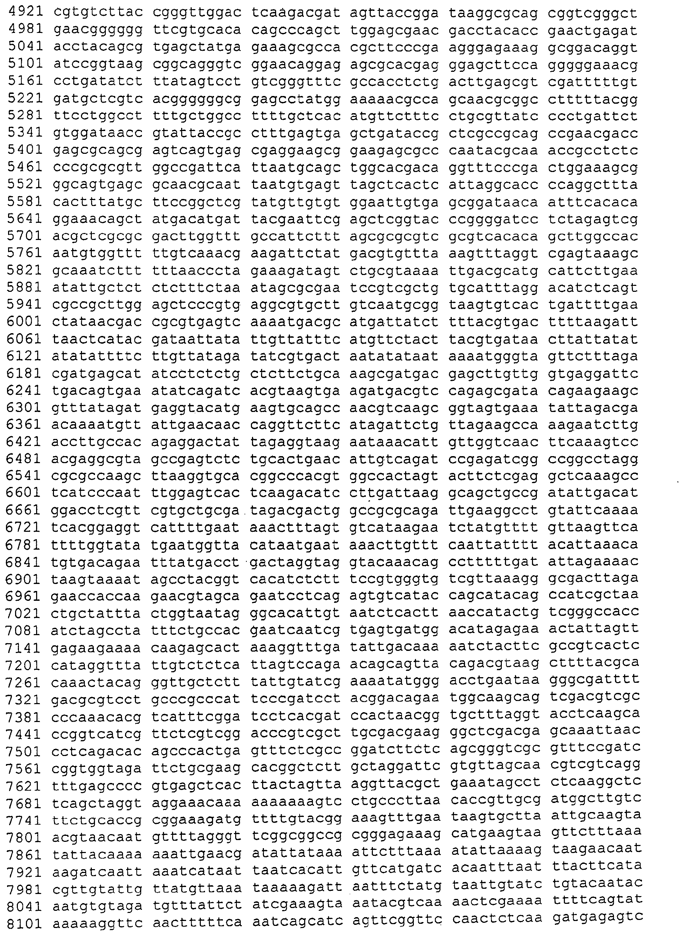

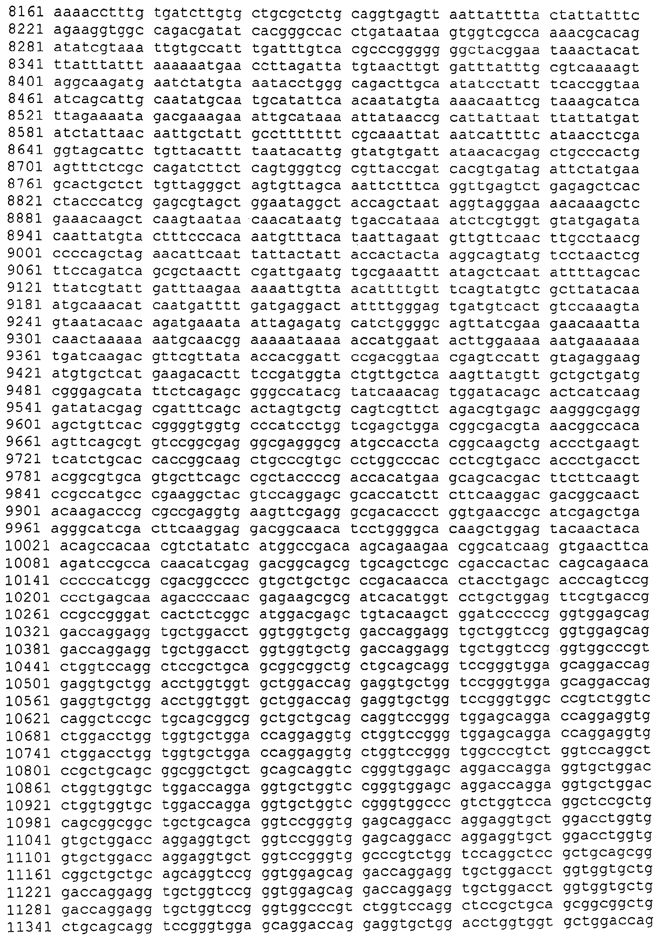

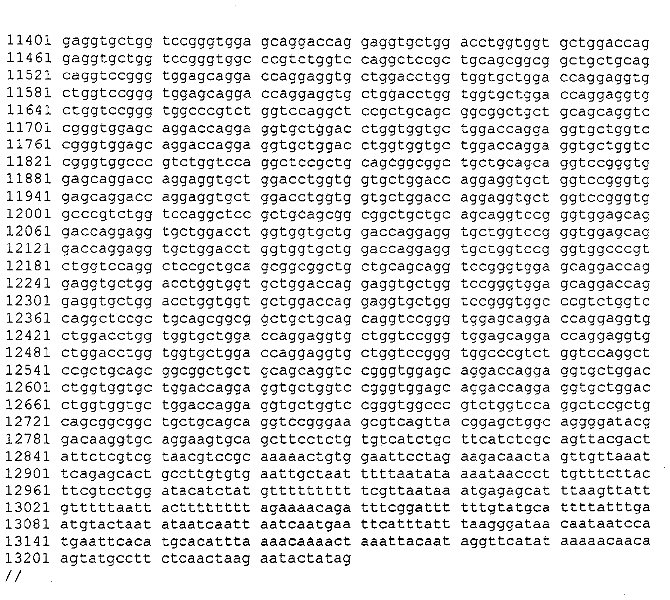

The present example is provided to describe the materials and methods/techniques employed in the creation of the transgenic silkworms, the general procedures employed in the creation of the genetic constructs employed, as well as reference tables used in the assessment of tensile strength of the transgenic spider silk fibers.
1. The gene sequences used. The gene sequences used are provided in the figures 13-16 provided herein. Variations of these are also envisioned as part of the present invention, as it is contemplated that shorter and/or longer versions of these sequences may be employed having conservative substitutions, for example, with substantially the same chimeric spider silk protein properties.
2. The chimeric spider silk proteins and the fibers obtained with these chimeric silk proteins will be assessed for tensile strength. Table 1 provides a general reference against with the chimeric spider silk fibers will be assessed. The chimeric spider silk fibers of the present invention were found to posses tensile and other mechanical strength characteristics similar to those of native spider silk.

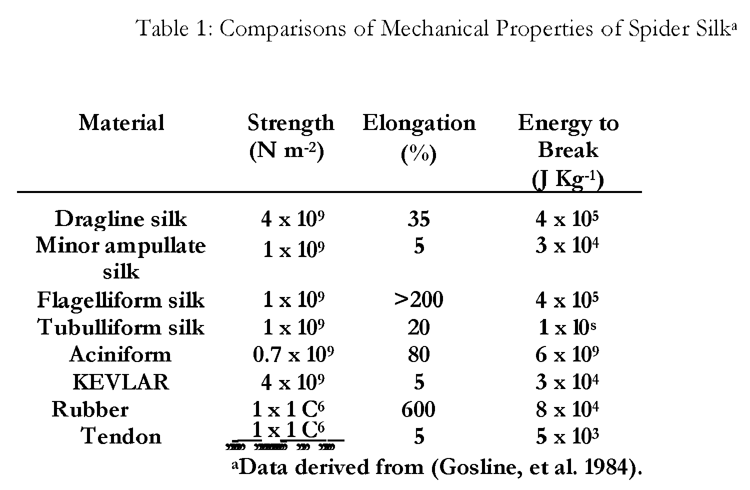

### Example 2Analysis of the tensile strength properties of individual transformed silkworm silks:

Transgenic silkworm silks were analyzed for the presence of the spider silk chimeric protein by western blotting of both the silkworm silk gland protein contents and the silk fibers from transgenic silkworm cocoons using a spider silk- specific antibody. In both cases transgenic silkworms were verified as producing the chimeric proteins, and differential extraction studies showed that these proteins were integral components of the transgenic silk fibers of their cocoons. Furthermore, expression of each of the chimeric green fluorescent protein fusions was apparent in both silk glands and fibers by direct examination of the silk glands or silk fibers using a fluorescent dissecting microscope. In most cases the amount of fluorescent protein in the fibers was high enough to be visualized by the green color the cocoons under normal lighting.

Table 2 shows an analysis of transgenic silks produced from individual transgenic silkworms. These analyses definitely show that the transgenic lines transformed with the Spider-4 or Spider-6 constructs produce chimeric spider silk/silkworm fibers with improved strengths compared to silk fibers from the untransformed silkworms. Significantly, these fibers are in some cases nearly twice as strong as the native silk. A two-fold improvement in the strength of a silkworm/spider silk chimeric fiber approximates the improvement deemed necessary to make silkworm silk as strong and flexible as spider silk. Thus, these results prove that that the silkworm may be genetically engineered to produce a chimeric spider silk/silkworm fiber that can compete favorably with native spider silk by using *piggyBac* vectors encoding specified strength and/or flexibility domains of spider silks to construct Bombyx/spider silk chimeric proteins.

**Table 2 : Analysis of tensile strengths for transgenic silkworm fibers compared to non-transformed pnd-wl and a commercial silkworm strain.**

| Table 2: Analysis of tensile strengths for transgenic silkworm fibers compared to non-transformed pnd-wl and a commercial silkworm strain. | | | | | |
|---|---|---|---|---|---|
| Sample No. | Silkworm lines | compensated tensile strength (N) | CGS unit converted tensile strength (dyn/21 denier) | CGS unit converted tensile strength (dyn/denier) | Fold Inaprovement Over pnd-w 1 |
| 1 | pnd -w1 control | 0.531 | 53131.1 | 2530.1 | 1 |
| 2 | P6+0 | 0.809 | 80947.7 | 3854.7 | 1.52 |
| 3 | P6+1 | 0.552 | 55155.2 | 2626.4 | 1.03 |
| 4 | P6+3 | 0.542 | 54218.2 | 2581.8 | 1.02 |
| 5 | P6+4 | 0.815 | 81496.7 | 3880.8 | 1.53 |
| 6 | P6+5 | 0.656 | 65594.1 | 3123.5 | 1.23 |
| 7 | P4+1 | 0.965 | 96460.6 | 4593.4 | 1.82 |
| 8 | P4+3 | 0.630 | 63000.0 | 3000.0 | 1.18 |
| 9 | Korean commercial | 0.676 | 67584.5 | 3218.3 | 1.27 |

### Example 3 - Silkworm Chimeric Gene Expression Cassettes and vi22vBac Vectors for Chimeric Snider Silk/Silkworm Protein Expression in Transgenic Silkworms

The present example is provided to demonstrate the utility and scope of the present invention in providing a vast variety of silkworm chimeric spider silk gene expression cassettes. The present example also demonstrates the completion of *piggyBac* vectors shown to successfully transform silk worms, and result in the successful production of commercially useful chimeric spider silk proteins suitable for the production of fibers of commercially useful lengths in manufacturing.

The expression cassettes.

Several variations on the basic expression cassettes shown below were constructed. These constructs reflect an assembly of constructs designed to express fibroin heavy chain (fhc)-spider silk chimeras, in which the synthetic spider silk protein sequence is flanked by N- and C-terminal fragments of the *B. mori* fhc protein. In this regard, several variations on a basic *Bombyx mori* silk fibroin heavy chain expression cassette shown in Figure 5 were constructed. The design involves the assembly of constructs designed to express fibroin heavy chain (fhc)-spider silk chimeras, in which the synthetic spider silk protein sequence is flanked by N- and C-terminal fragments of the *B. mori* fhc protein. The functionally relevant genetic elements in each expression cassette, from left to right, include: the major promoter, upstream enhancer element (UEE), basal promoter, and N-terminal domain (NTD) from the *B. mori* fhc gene, followed by various synthetic spider silk protein sequences (see below) positioned in-frame with the translational initiation site located upstream in the NTD, followed by the fhc C-terminal domain (CTD), which includes translational termination and RNA polyadenylation sites.

There are eight different versions of the expression cassette pictured in Figure 5, which encode four different synthetic spider silk/silworm proteins with or without EGFP inserted in-frame between the NTD and spider silk sequences. These sequences have been designated as "Spider 2 (reference example)", "Spider 4 (reference example)", "Spider 6", and "Spider 8 (reference example)" and they are defined as follows:
a) Spider 2: 7,104 bp, consisting of (A4S8)24. A1 indicates 4 copies of the putative flagelliform silk elastic motif (GPGGA); hence A4 indicates 16 copies of this same sequence. S8 indicates the putative dragline silk strength motif [GGPSGPGS(A)8], also described as the "linker-polyalanine" sequence. Approximate size of GFP(Green Florescent Protein) fusion protein is 161.9 + 50.4 = 212.3 Kd.
   Spider 4: 7,386 bp, consisting of (A2S8)x42. A2 indicates 8 copies of the putative flagelliform silk elastic motif (GPGGA). S8 indicates the putative dragline silk strength motif [GGPSGPGS(A)8], as above. Approximate size of GFP fusion protein is 169.4 + 50.4 = 219.8 Kd.
b) Spider 6: 2,462 bp, consisting of (A2S8)x14. A2 indicates 8 copies of the elastic motif (GPGGA) and S8 indicates the strength motif [GGPSGPGS(A)8], as above. Approximate size of GFP fusion protein is 56.4 + 50.4 = 106.8 Kd.
c) Spider 8 : 4,924 bp, consisting of (A2S8)x28. A2 indicates 8 copies of the elastic motif (GPGGA) and S8 indicates the strength motif [GGPSGPGS(A)8], as above. Approximate size of GFP fusion protein is 112.8 + 50.4 = 163.2 Kd.

The sizes of NTD exon I & II (1625 + 15161); eGFP (27135); CTD (6470) = 50,391 Kd.

### Example 4 - Subcloning the Expression Cassettes into piggyBac

Each of the eight different versions of the expression cassette pictured in Figure 5 (and described in Example 3) above were excised from a parent plasmid using Ascl and Fsel and subcloned into the corresponding sites of pBAC[3xP3-DSRedaf]. A map of this *piggyBac* vector is shown in Figure 6.

All *the piggyBac* vectors described above, with and without EGFP, were tested by PCR for the individual components and displayed the expected sized products.

Each of *the piggyBac* vectors encoding spider silk proteins fused to EGFP were functionally assessed by assaying their ability to induce EGFP expression in *B. mori* silk glands. Briefly, silk glands were removed from silkworms and a particle gun was used to bombard the glands with tungsten particles coated with *the piggyBac* DNA (or controls). The bombarded tissue was then cultured in Grace's medium in culture dishes and a dissecting microscope equipped for EGFP fluorescence available in a colleague's lab was used to examine the silk glands for EGFP expression two and three days later. Each vector was shown to induce EGFP fluorescence.

The set of *fourpiggyBac* vectors encoding Spider 4 and 6 with and without an EGFP insertion were used to produce transgenic silkworms.

### Example 5 - Isolation of Transgenic Silkworms.

Generally, silkworm transformation involves introducing a mixture of the *piggyBac* vector and a helper plasmid, encoding *the piggyBac* transposase, into pre-blastoderm embryos by microinjecting silkworm eggs. Blastoderm formation does not occur for as long as 4 h after eggs are laid. Thus, collection and injection of embryos can be done at room temperature over a relatively long time period. The technical hurdle for microinjection is the need to breach the egg chorion, which poses a hard barrier. Tamura and coworkers perfected the microinjection technique for silkworms by piercing the chorion with a sharp tungsten needle and then precisely introducing a glass capillary injection needle into the resulting hole. This is now a relatively routine procedure, accomplished with an eppendorf robotic needle manipulator calibrated to puncture the chorion, remove the tungsten needle, insert the glass capillary, and inject the DNA solution. The eggs are then re-sealed using a small drop of Krazy glue and maintained under normal rearing conditions of 28 degrees C and 70% humidity until the larvae hatch. The surviving injected insects are then mated to generate F1 generation embryos for the subsequent identification of putative transformants, based an expression of the DS-Red eye marker. Putative male and female transformants identified by this method are then mated to produce homozygous lineages for more detailed genetic analyses.

Specifically, silkworm transformation for the current project involved injecting a mixture of *the piggyBac* vector and helper plasmid DNAs into eggs of a clear cuticle silkworm mutant, *Bombyx mori* pnd-wl. This mutant silkworm is described by Tamura, et al. 2000, which reference is specifically incorporated herein by reference. This mutant has a melanization deficiency that makes screening using fluorescent genes much easier. Once red-eyed, putative F1 transformants were identified, homozygous lineages were established and bona fide transformants were confirmed using Western blotting of silk gland proteins and harvested cocoon silk.

### Example 6 - Analysis of Chimeric Spider silk/Silkworm Production bv Transgenic Silkworms

Transgenic silkworm silks were analyzed for the presence of the spider silk chimeric protein by Western blotting of both the silkworm silk gland protein contents and the silk fibers from transgenic silkworm cocoons using a spider silk- specific antibody. In both cases transgenic silkworms were verified as producing the chimericproteins, and differential extraction experiments showed that these proteins were integral components of the transgenic silk fibers of their cocoons.

Furthermore, expression of each of the chimeric green fluorescent protein fusions was apparent in both silk glands and fibers by direct examination of the silk glands or silk fibers using a fluorescent dissecting microscope. (Figure 7). In most cases the amount of fluorescent protein in the fibers was high enough to be visualized by the green color the cocoons under normal lighting.

### Example 7 - piggyBac Vector Design

*piggyBac* was the vector of choice for this project because it can be used to efficiently transform silkworms^{4'11' 43}. The specific *piggyBac* vectors used in this project were designed to carry genes with several crucial features. As highlighted in Fig. 17, these included the *B. mori* fibroin heavy chain (fhc) promoter, which would target expression of the foreign spider silk protein to the posterior silk gland^{91' 92}, and an fhc enhancer, which would increase expression levels and facilitate assembly of the foreign silk protein into fibers⁹³. *The piggyBac* vectors also encoded A2S8₁₄ (Fig. 17A), a relatively large, synthetic spider silk protein with both elastic (GPGGA)₈ and strength (linker-alanineg) motifs. The synthetic spider silk protein sequence was embedded within sequences encoding *N-* and C-terminal domains of the *Bombyx mori* fhc protein (Figs. 17B-17C). This chimeric silkworm/spider silk design had been used previously to direct incorporation of foreign proteins into nascent, endogenous silk fibers in the *B. mori* silk gland and produce composite silk fibers^{91. 92}.

One of *the piggyBac* vectors constructed in this study encoded the chimeric silkwomi/spider silk protein alone (Fig. 17B), while the other encoded this same protein with an N-terminal enhanced green fluorescent protein (EGFP) tag (Fig. 17C). The latter construct facilitated the analysis of silk fibers produced by transformed offspring and also was used for preliminary *ex vivo* silk gland bombardment assays to examine chimeric spider silk protein expression in silk glands, as described in herein.

### Methods:

Several gene fragments were isolated by polymerase chain reactions (PCR) with genomic DNA isolated from the silk glands of *Bombyx mori* strain P50/Daizo and the gene-specific primers shown in Figure 17. These fragments included the fhc major promoter and upstream enhancer element (MP-UEE), two versions of the fhc basal promoter (BP) and iV-terminal domain (NTD; exon 1/intron 1/exon 2) with different 5'- and 3'-flanking restriction sites, the fhc C-terminal domain (CTD; 3' coding sequence and polt' A signal), and EGFP. In each case, the amplification products were gel-purified, and DNA fragments of the expected sizes were excised and recovered. Subsequently, the fhc MP-UEE, fhc CTD, and EGFP fragments were cloned into pSLfa1180fa (pSL) (Y. Miao), the two different NTD fragments were cloned into pCR4-TOPO (Invitrogen Corporation, Carlsbad, CA), and *E. coli* transformants containing the correct amplification products were identified by restriction mapping and verified by sequencing.

These fragments were then used to assemble *the piggyBac* vectors used in this study as follows. The synthetic A2S8₁₄ spider silk sequence was excised from a pBluescript SKII+ plasmid precursor (F. Teule and R.V. Lewis) with *BamHl* and *BspEl,* gelpurified, recovered, and subcloned into the corresponding sites upstream of the CTD in the pSL intermediate plasmid described above. This step yielded a plasmid designated pSL-spider6-CTD. A *NotllBamHl* fragment was then excised from one of the pCR4- TOPO-NTD intermediate plasmids described above, gel-purified, recovered, and subcloned into the corresponding sites upstream of the spider 6-CTD sequence in pSLspider 6-CTD to produce pSL-NTD-spider 6-CTD. In parallel, a *NotUXbal* fragment was excised from the other pCR4-TOPO-NTD intermediate plasmid described above, gelpurified, recovered, and subcloned into the corresponding sites upstream of the EGFP amplimer in the pSL-EGFP intermediate plasmid described above. This produced a plasmid containing an NTD-EGFP fragment, which was excised with *Notl* and *BamHl* and subcloned into the corresponding sites upstream of the spider6-CTD sequences in pSL-spider 6-CTD. The MP-UEE fragment was then excised with *Sfi*/ and *Notl* from the pSL intermediate plasmid described above, gel-purified, recovered, and subcloned into the corresponding sites upstream of the NTD-spider 6-CTD and NTD-EGFP- spider 6-CTD sequences in the two different intermediate pSL plasmids described above. Finally, the completely assembled MP-UEE-NTD- A2S8 ₁₄-CTD or MP-UEE- NTD-EGFP-A2S8₁₄- CTD cassettes were excised with *Ascl* and *Fsel* from the respective final pSL plasmids and subcloned into the corresponding sites of pBAC[3XP3-DsRedaf] ⁹⁸. This final subcloning step yielded two separate piggyBac vectors that were designated spider 6 and spider 6-EGFP to denote the absence or presence of the EGFP marker. These vectors were used for *ex vivo* silk gland bombardment assays and silkworm transgenesis, as described below.

### Results:

The *ex vivo* assay results showed that *the piggyBac* vector encoding the GFP-tagged chimeric silkworm/spider silk protein induced green fluorescence in the posterior silk gland region. Immunoblotting assays with a GFP-specific antibody further demonstrated that the bombarded silk glands contained an immunoreactive protein with an apparent molecular weight (Mᵣ) of -116 kDa. Only slightly larger than expected (106 kDa), these results validated the basic design of the present *piggyBac* vectors and prompted the isolation of transgenic silkworms using these constructs.

### Example 8 - Transgenic Silkworm Isolation

*EachpiggyBac* vector was mixed with a plasmid encoding *the piggyBac* transposase and the mixtures were independently microinjected into eggs isolated from *Bombyx mori* pnd-wl ⁴³. This silkworm strain was used because it has a melanization deficiency resulting in a clear cuticle phenotype, which facilitated detection of the EGFP-tagged chimeric silkworm- spider silk protein in transformants. Putative F 1 transformants were initially identified by a red eye phenotype resulting from expression of DS-Red under the control of the neural- specific 3XP3 promoter²⁷ included in *each piggyBac* vector (Fig. 17D). These animals were used to establish several homozygous transgenic silkworm lineages, as described in Methods, which were designated spider 6 and spider 6-GFP, denoting *the piggyBac* vector used for their transformation.

### Methods:

### Ex-vivo silk gland bombardment assays

Live *Bombyx mori* strain pnd-wl silkworms entering the third day of fifth instar were sterilized by immersion in 70% ethanol for a few seconds and placed in 0.7% w/v NaCI. The entire silk glands were then aseptically dissected from each animal and transferred to Petri dishes containing Grace's medium supplemented with antibiotics, where they were held in advance of the DNA bombardment process. In parallel, tungsten microparticles (1.7 ium M-25 microcarriers; Bio-Rad Laboratories, Hercules, CA) were coated with DNA for bombardment, as follows. The microparticles were pre-treated according to the manufacturer's instructions and held in 3 mg/50 µi aliquots in 50% glycerol at -20°C. Just prior to each bombardment experiment, the 3 mg microparticle aliquots were coated with 5µg of the relevant *piggyBac* DNA in a maximum volume of 5 according to the manufacturer' s instructions. Some microparticle aliquots were coated with distilled water for use as DNA-negative controls. Each bombardment experiment included six replicates and each individual bombardment included one pair of intact silk glands. For bombardment, the glands were transferred from holding status in Grace's medium onto 90 mm Petri dishes containing 1% w/v sterile agar and the Petri dishes were placed in the Bio-Rad Biolistic® PDS-1000/He Particle Delivery System chamber. The chamber was evacuated to 20-22 in Hg and the silk glands were bombarded with the pre-coated tungsten microparticles using 1,100 psi of helium pressure at a distance of 6 cm from the particle source to the target tissues, as described previously ²⁶. After bombardment, the silk glands were placed in fresh Petri plates containing Grace's medium supplemented with 2X antibiotics and incubated at 28°C. Transient expression of the EGFP marker in the spider 6-GFP *piggyBac* vector was assessed by fluorescence microscopy at 48 and 72 hours post-bombardment. Images were taken with an Olympus FSX100 microscope at a magnification of 4.2X, a phase of 1/120 sec, and green fluorescence of 1/110 sec (capture). In addition, transient expression of the EGFP-tagged and untagged chimeric silkworm/spider silk proteins was assessed by immunoblotting bombarded silk gland extracts with EGFP- or spider silk-specific antisera, as described below.

### Silkworm transformation

Eggs were collected 1 hour after being laid by pnd-wl moths and arranged on a microscope slide. Vector and helper plasmids were resuspended in injection buffer (0.1 mM sodium phosphate, 5 mM KC1, pH 6.8) at a final concentration of 0.2 Kg/ul each, and 1-5 nl was injected into each preblastoderm silkworm embryo using an injection system consisting of a World Precision Instruments PV820 pressure regulator (USA), a Suruga Seiki M331 micromanipulator (Japan), and a Narishige HD-21 double pipette holder (Japan). The punctured eggs were sealed with Helping Hand Super Glue gel (The Faucet Queens, Inc., USA) and then placed in a growth chamber at 25°C and 70% humidity for embryo development. After hatching, the larvae were reared on an artificial diet (Nihon Nosan Co., Japan) and subsequent generations were obtained by mating siblings within the same line. Transgenic progeny were tentatively identified by the presence of the DsRed fluorescent eye marker using an Olympus SXZ12 microscope (Tokyo, Japan) with filters between 550 and 700 nm.

### Results:

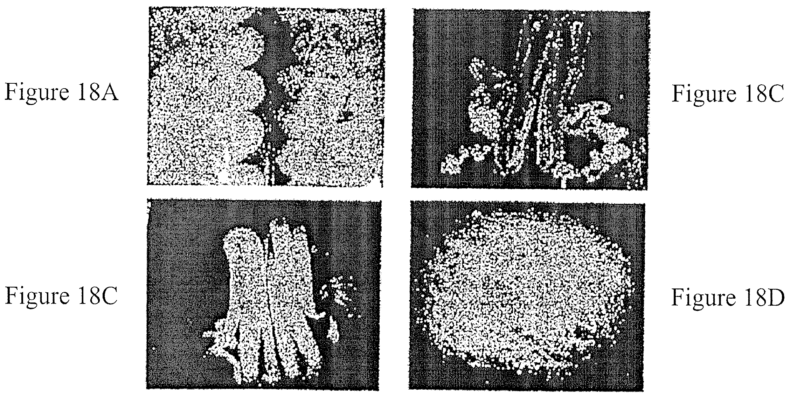

Even by visual inspection under white light, without specific EGFP excitation, EGFP expression was observed in cocoons produced by the spider 6-GFP transformants (Fig. 18A). Strong EGFP expression when silk glands (Figs. 18B-18C) and cocoons (Fig. 18D) from these animals were examined under a fluorescence microscope was also observed. The cocoons appeared to include at least some silk fibers with integrated EGFP signals. Expression of the EGFP-tagged chimeric silkworm/spider silk proteins in the spider 6-GFP silk glands and cocoons was confirmed by immunoblotting silk gland and cocoon extracts with EGFP- and spider silk protein- specific antisera (Fig. 19). Similar results were obtained with spider 6 silk gland and cocoon extracts by immunoblotting with the spider silk protein-specific antiserum (Fig. 19). These results indicated that we had successfully isolated transgenic silkworms encoding EGFP-tagged or untagged forms of the chimeric silkworm/spider silk protein and that these proteins were associated with the silk fibers produced by those transgenic animals.

### Example 9 - Analysis of the Composite Silk Fibers

A sequential protein extraction approach was used to analyze the association of the chimeric silkworm/spider silk proteins with the composite silk fibers produced by the transgenic silkworms. After removing the loosely associated sericin layer, the degummed silk fibers were subjected to a series of increasingly harsh extractions, as described in Methods.

### Methods:

### Sequential extraction of silkworm cocoon proteins

Cocoons produced by the parental and transgenic silkworms were harvested and the sericin layer was removed by stirring the cocoons gently in 0.05% (w/v) Na₂C 0 ₃ for 15 minutes at 85°C with a material: solvent ratio of 1:50 (w/v)⁴⁰. The degummed silk was removed from the bath and washed twice with hot (50-60°C) water with careful stirring and the same material : solvent ratio. The degummed silk fibers were then lyophilized and weighed to estimate the efficiency of sericin layer removal. The degummed fibers were used for a sequential protein extraction protocol, with rotation on a mixing wheel to ensure constant agitation, as follows. Thirty mg of the degummed silk fibers were treated with 1 ml of phosphate buffered sahne (PBS; 137 mM NaCl, 2.7 mM KC1, 10 mM Na₂P0₄, 1.8 mM KH₂P0₄) for 16 hours at 4°C. The material was separated into insoluble and soluble fractions by centrifugation, the supernatant was removed and held at -20°C as the PBSsoluble fraction, and the pellet was subjected to the next extraction. This pellet was resuspended in 1 ml of 2% (w/v) SDS and incubated for 16 hours at room temperature. Again, the material was separated into insoluble and soluble fractions by centrifugation, the supernatant was removed and held at -20°C as the SDS-soluble fraction, and the pellet was subjected to the next extraction. This pellet was resuspended in 1 ml of 9 M LiSCN containing 2% (v/v) B-mercaptoethanol and incubated for 16-48 hours at room temperature. After centrifugation, the supernatant was held at -20°C as the 9 M LiSCN/BME- soluble fraction. The final pellet obtained at this step was resuspended in 1 ml of 16 M LiSCN containing 5% (v/v) BME and incubated for about an hour at room temperature. This resulted in complete dissolution and produced the final extract, which was held as the 16 M LiSCN/BME-soluble fraction at -20C until the immunoblotting assays were performed.

### Analysis of silk proteins

Silk glands from the *ex vivo* bombardment assays and also from the untreated parental and transgenic silkworms were homogenized an ice in sodium phosphate buffer (30 mM Na₂P0₄, pH 7.4) containing 1% (w/v) SDS and 5 M urea, then clarified for 5 minutes at 13,500 rpm in a microcentrifuge at 4°C. The supernatants were harvested as silk gland extracts and these extracts, as well as the sequential cocoon extracts described above were diluted 4X with 10 mM Tris-HCl/2% SDS/5% BME buffer and samples containing -90 p,g of total protein were mixed 1:1 with SDS-PAGE loading buffer, boiled at 95°C for 5 minutes, and loaded onto 4-20% gradient gels (Pierce Protein Products; Rockford, IL). After separation, proteins were transferred from the gels to PVDF membranes (ImmobilonTM; Millipore, Billerica, MA) using a Bio-Rad transfer cell, according to the manufacturers' instructions. Immunodetection was performed using a spider silk protein specific polyclonal rabbit antiserum produced against the *Nephila elavipes* flagelliform silk-like A2 peptide (GenScript Corporation, Piscataway, NJ) or a commercial EGFPspecific mouse monoclonal antibody (Living Colors® GFP, Clontech Laboratories, Mountain View, CA) as the primary antibodies. The secondary antibodies were goat antirabbit IgG-HRP (Promega Corporation, Madison, WI) or goat anti-Mouse IgG H+L HRP conjugate (EMD Chemicals, Gibbstown, NJ), respectively. All antibodies were used at 1:10,000 dilutions in a standard blocking buffer (lx PBST/0.05% nonfat dry milk) and antibody-antigen reactions were visualized by chemiluminescence using a commercial kit (ECLTM Western Blotting Detection Reagents; GE healthcare).

### Results:

After each step in this procedure, the soluble and insoluble fractions were separated by centrifugation, the soluble fraction was held for immunoblotting, and the insoluble fraction was used for the next extraction. The final extraction solvent completely dissolved the remaining silk fibers. The immunoblotting controls verified that the spider silk protein- specific antiserum did not recognize any proteins in pnd-wl silk fibers (Fig. 19B, lanes 3-6), but recognized the chimeric silkwoina/A2S8 ¹⁴ spider silk protein produced in *E. coli* (Fig. 19B, lane 2). Sequential extraction of degummed cocoons from the transgenic animals using sahne (Fig. 19B, lanes 8 and 13), SDS (Fig. 19B, lanes 9 and 14), and 8M LiSCN/2% 13-mercaptoethanol (Fig. 19B, lanes 10 and *15)* failed to release any detectable immunoreactive proteins. However, subsequent extraction of the residual silk fibers with 16M LiSCN/5% β-mercaptoethanol released an immunoreactive protein with a Mᵣ of -106 kDa from the residual spider 6 (Fig. 19, lane 11) and two immunoreactive proteins with Mᵣs of -130 and -110 kDa from the residual spider 6-GFP fibers (Fig. 19, lane 16). All of these proteins were larger than expected (78 kDa and 106 kDa for spider 6 and spider 6-GFP, respectively). Possible explanations for these differences include transcriptional/translational 'stuttering' due to the highly repetitive nature of the spider silk sequences, anomalous migration of the protein products an SDS-PAGE, and/or post-translational modifications of the chimeric silkworm/spider silk proteins. The chimeric silkwoina/A2S8₁₄ spider silk protein produced in *E. coli,* which was the positive control for immunoblotting, also had a larger Mᵣ (-75 kDa) than expected (60 kDa). The 16M LiSCN/5% B-mercaptoethanol extracts from the degummed cocoons of both transgenic silkworm lines also included immunoreactive smears with Mᵣs from -40 to -75 kDa, possibly reflecting degradation of the chimeric silkworm/spider silk proteins and/or premature translational terminations. Irrespective of the sizes of the transgene products or the reasons for their appearance, the sequential extraction results clearly demonstrated that the transgenic silkworms provided as described here expressed chimeric silkworm/spider silk proteins that were extremely stably incorporated into composite silk fibers.

### Example 10 - Mechanical Properties of Composite Silk Fibers

The mechanical properties of degummed native and composite silk fibers of the composite silk fibers produced by the transgenic silkworms is described here.

The methods by which the composite silk fibers were prepared for testing, and how the testing was conducted, is presented below in Methods.

### Methods:

The degummed silkworm silk fibers used for mechanical testing had initial lengths (L₀) of 19 mm. Single fiber testing was performed at ambient conditions (2022°C and 19-22% humidity) using an MTS Synergie 100 system (MTS Systems Corporation, Eden Prairie MN) mounted with both a standard 50 N cell and a custom-made 10 g load cell (Transducer Techniques, Temecula CA). The mechanical data (load and elongation) were recorded from both load cells with TestWorks® 4.05 software (MTS Systems Corporation, Eden Prairie, MN) at a strain rate of 5 mm/min and frequency of 250 MHz, which allowed for the calculation of stress and strain values. The stress/strain curves from the data set gathered for each fiber were plotted using MATLAB (Version 7.1) to determine toughness (or energy to break), Young's Modulus (initial stiffness), maximum stress, and maximum extension (=maximum % strain).

### Results:

The results demonstrated that degummed composite fibers containing either the EGFP-tagged or untagged chimeric silkworm/spider silk proteins had significantly greater extensibility and slightly improved strength and stiffness than the native fibers from pnd-wl silkworms (Table 3 and Fig. 20). Table 3 : The mechanical properties of 12-15 silk fibers produced by the parental and transgenic silkworms were measured under precisely matched conditions of temperature, humidity, and testing speeds and the average values and standard deviations are presented in the Table. The average mechanical properties of spider *(Nephila elavipes)* dragline silk fiber determined in parallel under the exact same conditions are included for comparison.

**Table 3: Mechanical Properties of Degummed Native and Composite Silk Fibers**

| Mechanical Pf operty | Pnd-w1 | | Spider 6 | | Spider 6-GFP (line1) | | Spider 6-GFP (line4) | | Dragline (Spider) Avg |
|---|---|---|---|---|---|---|---|---|---|
| | Avg | SD | Avg | SD | Avg | SD | Avg | SD | |
| Max Stress (MPa) | 198.0 | 28.1 | 315.3 | 65.8 | 281.9 | 57.7 | 338.4 | 87.0 | 744.5 |
| Max Strain (%) | 22.0 | 5.8 | 31.8 | 5.2 | 32.5 | 4.3 | 31.1 | 4.5 | 30.6 |
| Toughness MJ/m³ | 32.0 | 10.0 | 71.7 | 13.9 | 68.9 | 16.2 | 77.2 | 29.5 | 138.7 |
| Young's modulus (MPa) | 3705.0 | 999.6 | 5266.8 | 1656.5 | 4860.9 | 1269.2 | 5498.1 | 1181.2 | 9267.7 |

| | | | | | | | | | |
|---|---|---|---|---|---|---|---|---|---|
| the mechanical properties of 12-15 silk fibers produced by the parental and transgenic silkworms were measured and the average values and standard deviations are presented in the Table. The optimal mechanical properties of spider *(Nephila clavipes)* dragline silk fiber determined under the same conditions are included for comparison. | | | | | | | | | |

Thus, these composite fibers are tougher than the native silkworm silk fibers. The mechanical properties of the composite silks produced by the transgenic animals were more variable than those of native fibers produced by the parental strain. In addition, the composite fibers produced by two different spider 6-GFP lines had similar extensibility, but different tensile strengths. The variations observed in the mechanical properties of composite silk fibers within an individual transgenic line and the line-to-line variation may reflect heterogeneity in the composite fibers, the heterogeneity may be due to differences in the chimeric silkworm/spider silk protein ratios and/or the localization of these proteins along the fiber. One can see evidence of heterogeneity in the composite fibers in Fig. 18D. A comparison of the best mechanical performances observed for the composite fibers from the transgenic silkworms, native fibers from the parental silkworm, and a representative dragline spider silk fiber is shown in Fig. 20. The results showed that all of the composite fibers were tougher than the native silk fiber from pnd-wl silkworms. Furthermore, the composite fiber from the transgenic spider 6-GFP line 4 silkworms was even tougher than a native spider dragline silk fiber tested under the same conditions. These results demonstrate that the incorporation of chimeric silkworm/spider silk proteins can significantly improve the mechanical properties of composite silk fibers produced using the transgenic silkworm platform.

The best mechanical performances measured with native silkworm (pnd-wl) and spider (N. *clavipes* dragline) silk fibers are compared to those obtained with the composite silk fibers produced by transgenic silkworms. All fibers were tested under the same conditions. The toughest values are: silkworm pnd-wl (blue line, 43.9 MJ/m3); spider 6 line 7 (orange line, 86.3 MJ/m3); spider 6-GFP line 1 (dark green line, 98.2 MJ/m3), spider 6-GFP line 4 (light green line, 167.2 MJ/m3); and N. clavipes dragline (red line, 138.7 MJ/m3). (See Table 3).

### Example 11 - Stably Incorporated Chimeric Silkworm/Spider Silk Protein-Containing Composite Fibers

Spider silks have enormous use as biomaterials for mang different applications. Previously, serious obstacles to spider farming crippled such as a natural manufacturing effort. The need to develop an effective biotechnological approach for spider silk fiber production is presented in the platform provided in the present disclosure. While other platforms have been described for use in the production of recombinant spider silk proteins, it has been difficult to efficiently process these proteins into useful fibers. The requirement to manufacture fibers, not just proteins, positions the silkworm as a qualified platform for this particular biotechnological application.

A transgenic silkworm engineered to produce a spider silk protein was isolated using *apiggyBac* vector encoding a native *Nephila clavipes* major ampullate spidroin- 1 silk protein under the transcriptional control of a *Bombyx mori* sericin (Serl) promoter. The spidroin sequence was fused to a downstream sequence encoding a C-terminal fhc peptide. The transgenic silkworm isolated using this *piggyBac* construct produced cocoons containing the chimeric silkworm/spider silk protein, but this protein was only found in the loosely associated sericin layer. In contrast, the chimeric silkworm/spider silk protein produced by the presently disclosed transgenic silkworms was an integral component of composite fibers. The relatively Loose association of the chimeric silkworm/spider silk protein designed by others, may, among other things, reflect the absence of an N-terminal silkworm fhc domain. Alternatively, the use of the Serl promoter in *apiggyBac* vector may, among other things, be inconsistent with proper fiber assembly, as this promoter is transcriptionally active in the middle silk gland, whereas the fhc, flc, and fhx promoters, which control expression of the fhc, fibroin light chain, and hexamerin proteins, respectively, are active in the posterior silk gland. The assembly of silkworm silk proteins into fibers is controlled, in part, by tight spatial and temporal regulation of silk gene expression. Thus, the presently disclosed vectors are engineered with the fhc promoter to drive accumulation of the chimeric silkworm/spider silk protein in the same place and at the same time as the native silk proteins, in order to facilitate stable integration of the chimeric protein into newly assembled, composite silk fibers. Others have described minor increases in the elasticity and tensile strength of fibers from the cocoons produced by some transgenic silkworms. However, the sericin layer was not removed prior to mechanical testing, and this degumming step is essential in the processing of cocoons for commercial silk fiber production. Thus, if cocoons had been processed in conventional fashion, the recombinant spider silk/silkworm protein would be removed and the resulting silk fibers would not be expected to have improved mechanical properties.

Transgenic silkworms producing spider silk proteins were reported as a relatively minor component of other studies, which focused on the regeneration of fibers from silk proteins dissolved in hexafluoro solvents. Nevertheless, this study described two transgenic silkworms produced *withpiggyBac* vectors encoding extremely short, synthetic, "silk-like" sequences from *Nephila elavipes* major ampullate spidroin-1 or flagelliform silk proteins. Both silk-like peptides were embedded within *N-* and C-terminal fhc domains. Mechanical testing showed that the silk fibers produced by these transgenic animals had slightly greater tensile strength (41-73 MPa), and no change in elasticity. These workers also report that the relatively small changes observed in the mechanical properties of their composite fibers reflected a low level of recombinant protein incorporation. It is also is possible that the specific spider silk-like peptide sequences used in those constructs and/or their small sizes may account, at least in part, for the relatively small changes in the mechanical properties of the composite fibers produced by those transgenic
silkworms.

The present transgenic silkworms and composite fibers are the first to yield transgenic silkworm lines that produce composite silk fibers containing stably integrated chimeric silkworm/spider silk proteins that significantly improve their mechanical properties. The composite spider silk/silkworm fiber produced by the present transgenic silkworm lines was even tougher than a native dragline spider silk fiber. Among other factors, this may at least in part be due to the use of the 2.4 kbp A2S8₁₄ synthetic spider silk sequence encoding repetitive flagelliform-like (GPGGA)₈ elastic and major ampullate spidroin-2 [linker-alanine8] crystalline motifs. This relatively large synthetic spider silk protein may be spun into fibers by extrusion after being produced in *E. coli,* indicating that it retained the native ability to assemble into fibers. However, this protein would be expressed in concert and would have to interact with the endogenous silkworm fhc, flc, and fhx proteins in order to be incorporated into silk fibers. Thus, the A2S8₁₄ spider silk sequence was embedded within *N*- and C-terminal fhc domains to direct the assembly process. Together with the ability of the fhc promoter to drive their expression in spatial and temporal proximity to the endogenous silkworm silk proteins, these features may at least in part account for the ability of the chimeric silkworm/spider silk proteins to participate in the assembly of composite silk fibers and contribute significantly to their mechanical properties.

### Example 12 - piggyBac Vector Constructs and PCR Amplification of Components of piggyBac Vectors

Several gene fragments were isolated by polymerase chain reactions with genomic DNA isolated from the silk glands of *Bombyx mori* strain P50/Daizo and the gene-specific primers shown in Table 4. These fragments included the fhc major promoter and upstream enhancer element (MP-UEE), two versions of the fhc basal promoter (BP) and iV-terminal domain (NTD; exon 1/intron 1/exon 2) with different 5'- and 3'-flanking restriction sites, the fhc C-terminal domain (CTD; 3' coding sequence and polt' A signal), and EGFP. In each case, the amplification products were gel-purified, and DNA fragments of the expected sizes were excised and recovered. Subsequently, the fhc MP-UEE, fhc CTD, and EGFP fragments were cloned into pSLfal 180fa, the two different NTD fragments were cloned into pCR4-TOPO (Invitrogen Corporation, Carlsbad, CA), and *E. coli* transformants containing the correct amplification products were identified by restriction mapping and verified by sequencing. These fragments were than used to assemble *thepiggyBac* vectors used in this study os follows. The synthetic A2S8₁₄ spider silk sequence was excised from a pBluescript SKII+ plasmid precursor with *BamHl* and *BspEL,* gel-purified, recovered, and subcloned into the corresponding sites upstream of the CTD in the pSL intermediate plasmid described above. This step yielded a plasmid designated pSL-spider6-CTD. A *Notl*/*BamH1* fragment was then excised from one of the pCR4-TOPO-NTD intermediate plasmids described above, gel-purified, recovered, and subcloned into the corresponding sites upstream of the spider 6-CTD sequence in pSL-spider 6-CTD to produce pSL-NTD-spider 6-CTD. In parallel, *allotVXbal* fragment was excised from the other pCR4-TOPO-NTD intermediate plasmid described above, gel-purified, recovered, and subcloned into the corresponding sites upstream of the EGFP amplimer in the pSL-EGFP intermediate plasmid described above. This produced a plasmid containing NTD-EGFP fragment, which was excised with *Notl* and *BamHl* and subcloned into the corresponding ites upstream of the spider6-CTD sequences in pSL-spider 6-CTD. The MP-UEE fragment was then excised with *Stil* and *Notl* from the pSL intermediate plasmid described above, gel-purified, recovered, and subcloned into the corresponding sites upstream of the NTD-spider 6-CTD and NTD-EGFP- spider 6-CTD sequences in the two different intermediate pSL plasmids described above. Finally, the completely assembled MP-UEE-NTD- A2S8i₄-CTD or MP-UEE- NTD-EGFP-A2S 814-CTD cassettes were excised with *AScl* and *Fsel* from the respective final pSL plasmids and subcloned into the corresponding sites of pBAC[3XP3-DsRedaf] (Horn, et al. (2002), Insect Biochem. Mol. Biol., 32:1221-1235). This final subcloning step yielded two separate *piggyBac* vectors that were designated spider 6 and spider 6-EGFP to denote the absence or presence of the EGFP marker. The following table provides a listing of some of the key components of *the piggyBac* vectors used.

| **Table 4** | | | | | | |
|---|---|---|---|---|---|---|
| | **Name** | **Sequence (5' to 3')** | **Restr Site(s) Added** | **Templa te DNA** | **Primer combination forPCRs** | **Amplification Products & Sizes** |
| 1 | Major pro (SP) | TAACTCGAGGCTCAAAGCCTCATCCCAATTTGGAG | 5'Xho I | | | Fhc Major **Promoter** |
| 2 | Major pro (ASP) | ATACCGCGGTGCAGAAGACAAGCCATCGCAACGGTG | 3' Sac II | | **1 & 2** | -5,000 to -3,844 (1,157 bp) |
| 3 | UEE (SP) | ATACCGCGGAAAGATGTTTTGTACGGAAAGTTTGAA | 5' Sac II | | **3 & 4** | **Fhc Enhancer** -1,659 to -1,590 (⁷⁰bp) |
| 4 | UEE (ASP) | TTAGCGGCCGCCGAACCCTAAAACATTGTTACGTTACGTTACTTG | 3' Not I | B.mori genomic DNA | | |
| 5 | Fhc pro+NTD (SP) | TAAGCGGCCGCGGGAGAAAGCATGAAGTAAGTTCTTTAAATATTACAAAAA | 5' Not I | | **5 & 6 5 & 7** (-) (+) | **Spider 6** EGFP (-) or (+) **expression cassettes** |
| 6 | Fhc Pro + NTD (ASP) | ATAGGATCCACGACTGCAGCACTAGTGCTGCTGAAATCGC | 3' Bam HI | | | Fhc Basal Promoter & 5' cds |
| 7 | Fhc Pro + NTD (ASP for EGFP) | ATATCTAGAACGACTGCAGCACTAGTGCTGCTGAAATCGC | 3' Xba I | | | **-F62,118** to **+63,816** (1,744 bp) |
| 8 | EGFP (SP) | CAATCTAGACGTGAGCAAGGGCGAGGAGCTGTTCACC | 5' Xba I | pEGFP-N1 plasmid DNA | **8 & 9** | EGFP (720 bp) |
| 9 | EGFP (ASP) | TAAGGATCCAGCTTGTACAGCTCGTCCATGCCGAGAG | 3' Bam HI | | | |
| 10 | FHc CTD (SP) | ATACCCGGGAAGCGTCAGTTACGGAGCTGGCAG | 5' Xma 1 | B .mori genomic DNA | 10 & II | Fhc 3'cds & **poly-A signal** +79,021 to +79,500 (480 bp) |
| 11 | Fhc CTD (ASP) | CAAGCTGACTATAGTATTCTTAGTTGAGAAGGCATAC | 3' Sal I | | | |

### Example 13 - masp Cloning

The present example demonstrates the utility of the present invention by providing genetic constructs that contain the NTD region within a plasmid, and in particular, the pXLBacII ECFP plasmid.

Potential positive clones containing the NTD region with the pXLBacII ECFP plasmid are shown by colony screening with PCR.

The genetic construct masp for the pXLBacII-ECFP NTD CTD maspX 16 (10,458 bp) (Figure 12A) and pXLBacII-ECFP NTD CTD maspX24 (11,250 bp) (Figure 12B) were created.

### BIBLIOGRAPHY

1. Berghammer, A., Bucher, G., Maderspacher, F., and Klingler, M. (1999), Dev. Genes Evol., 209: 382-389.
2. Birnboim, H. C , and Doly, J. (1979), Nucl. Acids Res., 7: 1513-1523.
3. Brooks, A. E., Creager, M., and Lewis, R. V. (2005), Altering the Mechanics of Spider Silk Through Methanol Post-spin Draw. In "Biomedical Sciences Instrumentation", Vol. 41, pp. 1-6.
4. Cary, L. C , et al. (1989), Virology, 172: 156-169.
5. Choudary, P. V., Kamita, S. G., and Maeda, S. (1995), Expression of foreign genes in Bombyx mori larvae using baculovirus vectors. In "Baculovirus expression protocols" (C. D. Richardson, Ed.), Vol. 39, pp. 243-264. Humana Press, Clifton, N.J.
6. Colgin, M., and Lewis, R. V. (1998), Protein Science, 7: 667-672.
7. Denny, M. W. (1980), Symp. Soc. Exp. Biol., 34: 247-272.
8. Dooling, D. (2005), Growing your own spare parts: NASA assists ligament replacement research.
9. Elick, T. A., Bauser, C. A., Principe, N. M., and Fraser, M. J., Jr. (1996), Genetica, 97: 127-139.
10. Fahnestock, S. R., and Bedzyk, L. A. (1997), Appl. Microbiol. Biotechnol., 47: 33-39.
11. Fraser, M. J. (2000), The TTAA-specific family of transposable elements. In "Insect Transgenesis: Methods and Applications." (A. A. James, and A. H. Handler, Eds.). CRC Press, Orlando.
12. Fraser, M. J., Brusca, J. S., Smith, G. E., and Summers, M. D. (1985), Virology, 145: 356-361.
13. Fraser, M. J., Cary, L., Boonvisudhi, K., and Wang, H. G. (1995), Virology, 211: 397-407.
14. Fraser, M. J., Smith, G. E., and Summers, M. D. (1983), J. Virol., 47: 287-300.
15. Gatesy, J., Hayashi, C , Motriuk, D., Woods, J., and Lewis, R. (2001), Science, 291: 2603-2605.
16. Gosline, J. M., Denny, M. W., and DeMont, M. E. (1984), Nature 309: 551-552.
17. Handler, A. M., and Gomez, S. P. (1995), Mol. Gen. Genet., 247: 399-408.
18. Handler, A. M., and Gomez, S. P. (1996), Genetics, 143: 1339-1347.
19. Handler, A. M., and Harrell, R. A., 2nd (1999), Insect Mol. Biol., 8: 449-457.
20. Handler, A. M., and Harrell, R. A., 2nd (2001), Insect Biochem. Mol. Biol., 31: 199-205.
21. Handler, A. M., McCombs, S. D., Fraser, M. J., and Saul, S. H. (1998), Proc. Natl. Acad. Sei. U. S. A., 95: 7520-7525.
22. Hayashi, C. Y., and Lewis, R. V. (2000), Science, 287: 1477-1479.
23. Hayashi, C. Y., Shipley, N. H., and Lewis, R. V. (1999), Int. J. Biol. Macromol., 24: 271-275.
24. Hinman, M. B., and Lewis, R. V. (1992), J. Biol. Chem., 267: 19320-19324.
25. Holland, C , Terry, A. E., Porter, D., and Vollrath, F. (2006), Nat. Mater., 5: 870-874.
26. Horard, B., Mange, A., Pelissier, B., and Couble, P. (1994), Insect Mol. Biol., 3: 261-265.
27. Horn, C , Jaunich, B., and Wimmer, E. A. (2000), Dev. Genes EvoL, 210: 623-629.
28. Huemmerich, D., et al. (2004), Curr., Biol. 14: 2070-2074.
29. Imamura, M., et al. (2003), Genetics, 165: 1329-1340.
30. Inoue, S., et al. (2005), Insect Biochem. Mol. Biol., 35: 51-59.
31. Inoue, S., et al. (2000), J. Biol. Chem., 275: 40517-40528.
32. Lazaris, A., et al. (2002), Science 295: 472-476.
33. Lewis, R. V., et al. (1996), Prot. Expr. Purif., 7: 400-406.
34. Lobo, N., Li, X., and Fraser, M. J., Jr. (1999), Mol. Gen. Genet., 261: 803-810.
35. Maeda, S., et al. (1985), Nature, 315: 592-594.
36. Mori, K., et al. (1995). J. Mol. Biol. 251: 217-228.
37. O'Brochta, D. A., Gomez, S. P., and Handler, A.M. (1991), Mol. Gen. Genet. 225: 387-394.
38. Peloquin, J. J., et al. (2000), Insect Mol. Biol., 9: 323-333.
39. Sambrook, J., Fritsch, E. F., and Maniatis, T. (1989), "Molecular Cloning: A Laboratory Manual." 2nd edition ed. Cold Spring Harbor Press, Cold Spring Harbor, New York.
40. Scheller, J., Guhrs, K. H., Grosse, F., and Conrad, U. (2001), Nat. BiotechnoL, 19: 573-577.
41. Southern, E. M. (1975), J. Mol. Biol., 98: 503-517.
42. Takei, F., et al. (1984), J. Cell Biol., 99: 2005-2010.
43. Tamura, T., et al. (2000), Nat. BiotechnoL, 18: 81-84.
44. Thibault, S . T., Luu, H. T., Vann, N., and Miller, T. A. (1999), Insect Mol. Biol., 8: 119-123.
45. Thomas, J. L., et al. (2002), Insect Biochem. Mol. Biol., 32, 247-253.
46. Tomita, M., et al. (2003), Nat. Biotechnol., 21: 52-56.
47. Towbin, H., et al. (1979), Proc. Natl. Acad. of Sci. U.S.A., 76: 4350-4354.
48. Urry, D. W. (2002), Philosophical Transactions of the Royal Society of London B 357:169-184.
49. Wang, H. G., and Fraser, M. J. (1993), Insect Mol. Biol., 1: 109-116.
50. Wang, H. H., Fraser, M. J., and Cary, L. C. (1989), Gene., 81: 97-108.
51. Wong Po Foo, C , et al. (2006), Appl. Phys. A., 82: 223-233.
52. Wurm, F. M. (2003), Nat. Biotechnol., 21: 34-35.
53. Xu, M., and Lewis, R. V. (1990), Proc. Natl. Acad. Sci. U.S.A., 87: 7120-7124.
54. Yamao, M., et al. (1999), Genes. Dev., 13: 511-516.
55. Yun, et at (2001), "Altering fibrin heavy chain gene of silkworm Bombyxmori by homologous recombination," Shengwu Huaxe yu Shrngwu Wuli Xuebao 33(1): 112-116.
56. GenBank Acc. No. AF226688, Zhou, et al. "Bombyx mori fibroin heavy chain Fib-H (fib-H) gene, complete cds.," US Natl. Library of Medicine, Bethesda, MD, USA, Jun. 19,2000.
57. Zhao, et al. (2001), Acta Biochimica et Biophysica Sinica, 33(1): 112-116.
58. Zhang, et al. (1999), Acta Biochimica et Biophysica Sinica, 31(2): 119-123.
59. Zhou, C.Z., et al. (2000), Nucleic Acids Res., 28. (12): 2413-2419.
60. Tomita, M., et al. (2003), Nat. Biotechnol., 21 (1): 52-56.
61. Yoshizato, Katsutoshi, "A Proposal for Application of Recombinant Insects (Kumikactai Konchu Riyo Eno Teigen)", Sanshi Konchuken Shiryo, No. 28, pp. 9395.
62. Toshiki, et al. (2000), Nature Biotechnology, 16: 81-85.
63. Okano, et al. (2000), Journal of Interferon and Cytokine Research, 20: 1015-1022.
65. Xiao-Hui, et al.(2000), Acta Pharmacol. Sin., 21 (9): 797-801.
66. Ishihara, et al. (1999), Biochimica et Biophysica Acta, 1451: 48-58.
67. T. Tamura, "Construction and utilization of transgenic silkworm using transposon", Fiber Preprints, Japan, Vol. 56, No. 2, 2001, p. 3841. A. Yanai, et al. (2002), Research Journal of Food and Agriculture, 25 (2): 30-33.
68. T. Tamura, et al. (2000), Agriculture and Horticulture, 75 (8): 17-24.
69. Yoshizato, Katsutoshi (2001), "A Proposal for Application of Recombinant Insects (Kumikaetai Konchu Riyo Eno Teigen)", Sanshi Konchuken Shiryo, 28: 93 95.
70. United States Patent 7,674,882 - Kaplan, et al.
71. United States Patent 7,659, 112 - Hiramatsu, et al.
72. United States Patent 7,521,228 - Lewis, et al.
73. United States Patent 6,268, 169 - Fahnestock.
74. United States Patent 5,994,099 - Lewis.
75. United States Patent 5,989,894 - Lewis.
76. United States Patent 5,756,677 - Lewis
77. United States Patent 5,733,771 - Lewis.
78. Kluge, J.A., Rabotyagova, 0., Leisk, G.G. & Kaplan, D.L. (2008), Trends Biotechnol, 26:244-251.
79. Scheibel, T. (2004), Microb. Cell. Fact. 3, 14.
80. Macintosh, A.C., Kearns, V.R., Crawford, A. & Hatton, P.V. (2008), J. Tiss. Engr. Reg. Med., 2 :71-80.
81. Gosline, J.M., Guerette, P.A., Ortlepp, C.S. & Savage, K.N. (1999), J. Exp. Biol., 202 :3295-3303.
82. Lewis, R.V. (2006), Chem. Rev., 106 :3762-3774.
83. Hardy, J.G., L.M., R. & T.R. (2008), S., Polymer, 49:4309-4327.
84. Teule, F., et al. (2007), 1. Mat. Set, 42 :8974-8985.
85. Teule, F., et al. (2009), Nat. Protoc. 4:341-355.
86. Fahnestock, S.R. & Irwin, S.L. (1997), Appl. Microbiol. Biotechnol., 47 :23-32.
87. Fahnestock, S.R. & Bedzyk, L.A. (1997), Appl. Microbiol. Biotechnol., 47:33-39.
88. Zhang, Y., et al. (2008), Mol. Biol. Rep. 35:329-335.
89. Miao, Y., et al. (2006), Appl. Microbiol. Biotechnol., 71:192-199.
90. Kato, T., Kajikawa, M., Maenaka, K. & Park, E.Y. (2010), Appl. Microbiol. Biotechnol, 85:459-470.
91. Royer, C , et al. (2005), Transgenic Res., 14:463-472.
92. Kojima, K,. et al. (2007), Biosci. Biotechnol. Biochem. 71, 2943-2951.
93. Kurihara, H., Sezutsu, H., Tamura, T. & Yamada, K. (2007), Biochem. Biophys. Res. Commun., 355 :976-980.
94. Shimizu, K., et al. (2007), Insect Biochem. Mol. Biol, 37:713-725.
95. Yanagisawa, S., et al. (2007), Biomacromolecules, 8:3487-3492.
96. Wen, H., et al. (2010), Mol. Biol. Rep., 37:1815-1821.
97. Zhu, Z., et al. (2010), J. Biomater. Sci. olym. Ed., 21:395-41 1.
98. Sehnal, F. & Akai, H. (1990), Int. InsectMorph. Embryol., 19:79-132.
99. Horn, C , et al. (2002), Insect Biochem. Mol. Biol., 32:1221-1235.
100. Yamada, H., Nakao, H., Takasu, Y. & Tsubouchi, K. (2001), Mat. Sci. Engr. C, 14:,41- 46.
101. United States Patent 5,728,810 Lewis.

## Claims

1. A method of preparing a transgenic *Bombyx mori* silkworm capable of expressing a stable chimeric spider silk protein suitable for assembly into a spider silk composite fiber, said chimeric spider silk protein encoded by a sequence comprising a spider silk flexibility motif sequence and a spider silk strength motif sequence, said method comprising:
a.) inserting a piggyBac vector as defined in FIG. 15 or FIG. 16, each having a recombinant genetic cassette encoding a combination of a spider silk flexibility motif sequence and a spider silk strength motif sequence defined by 14 repeated subunits, each subunit having 8 copies of a putative flagelliform silk elastic motif GPGGA and a putative dragline silk strength motif GGPSGPGS(A)8, into a mutant *Bombyx mori* egg to provide injected *Bombyx mori* eggs;
b.) allowing the eggs to hatch under suitable incubation conditions to provide larvae;
c.) permitting the larvae to mature under suitable incubation conditions; and
d.) selecting transgenic *Bombyx mori* silkworm.

2. The method of claim 1 wherein the recombinant genetic cassette comprises the piggyBac vector defined in FIG. 16 encoding a green fluorescent protein (GFP) for selection of transgenic *Bombyx mori.*

3. A transgenic silkworm made according to the methods of claim 1 or claim 2, the transgenic silkworm having a recombinant chimeric gene encoding the sequence comprising the combination of spider silk elasticity motif sequences and the spider silk strength motif sequences,
wherein the combination is (A2S8)x14 such that A2 indicates 8 copies of a putative flagelliform silk elastic motif GPGGA and S8 indicates a putative dragline silk strength motif GGPSGPGS(A)8, and
wherein the transgenic silkworm is capable of producing a chimeric silkworm/spider silk protein from the recombinant chimeric gene, the chimeric silkworm/spider silk protein being suitable for the production of a chimeric spider silk/silkworm fiber having a tensile strength 2x greater than a native silkworm fiber.

4. A manufacturing method for the production of chimeric spider silk composite fiber comprising:
a.) preparing the transgenic silkworm of claim 3;
b.) allowing the transgenic silkworm to produce a cocoon under suitable physiological conditions native to the silkworm;
c.) collecting the cocoons and extracting chimeric spider silk fiber.

5. A genetic construct designed to express fibroin heavy chain (fhc) spider silk chimeras in which a synthetic spider silk protein is flanked by N- and C-terminal fragments of a *Bombyx mori* fhc protein, the genetic construct comprising, in a 5' to 3' direction:
a major promoter from the *B. mori* fhc gene;
an upstream enhancer element from the *B. mori* fhc gene;
a basal promoter from the *B. mori* fhc gene;
an N-terminal domain (NTD) from the *B. mori* fhc gene;
various synthetic spider silk protein sequences positioned in-frame with a translation initiation site located upstream in the NTD, wherein the various synthetic spider silk protein sequences comprise a chimeric spider silk protein having 14 repeated subunits, each subunit having 8 copies of a putative flagelliform silk elastic motif GPGGA and a putative dragline silk strength motif GGPSGPGS(A)8; and
a fhc C-terminal domain (CTD) from the *B. mori* fhc gene that includes translation termination and RNA polyadenylation sites.

6. The genetic construct of claim 5, wherein the various spider silk protein sequences comprises 14 repeated subunits, each subunit having 8 copies of the putative flagelliform silk elastic motif GPGGA and the putative dragline silk strength motif GGPSGPGS(A)8, and wherein the genetic construct further comprises an EGFP sequence upstream of said chimeric spider silk protein sequence.

7. The genetic construct of claim 5, wherein the genetic construct is the piggyBac vector as defined in FIG. 15 or FIG. 16.

## Patentansprüche

1. Verfahren zur Herstellung einer transgenen Bombyx mori-Seidenraupe, die in der Lage ist, ein stabiles chimäres Spinnenseidenprotein zu exprimieren, das für den Zusammenbau zu einer Spinnenseiden-Mischfaser geeignet ist, wobei das chimäre Spinnenseidenprotein durch eine Sequenz codiert wird, die eine Spinnenseiden-Flexibilitätsmotivsequenz und eine Spinnenseiden-Festigkeitsmotivsequenz umfasst, wobei das Verfahren umfasst:
a.) Einfügen eines piggyBac-Vektors wie in Fig. 15 oder Fig. 16 definiert, die jeweils eine rekombinante genetische Kassette aufweisen, die eine Kombination von einer Spinnenseiden-Flexibilitätsmotivsequenz und einer Spinnenseiden-Festigkeitsmotivsequenz definiert durch 14 wiederholte Untereinheiten codiert, wobei jede Untereinheit 8 Kopien eines putativen Flagelliform-Seiden-elastischen Motivs GPGGA und eines putativen Dragline-Seiden-Festigkeitsmotivs GGPSGPGS(A)8 aufweist, in ein Mutanten-Bombyx mori-Ei, um injizierte Bombyx mori-Eier bereitzustellen;
b.) Ausbrüten lassen der Eier unter geeigneten Inkubationsbedingungen, um Larven zu erhalten;
c.) es den Larven ermöglichen, sich unter geeigneten Inkubationsbedingungen zu entwickeln; und
d.) Selektieren von transgener Bombyx mori-Seidenraupe.

2. Verfahren nach Anspruch 1, wobei die rekombinante genetische Kassette den in Fig. 16 definierten piggyBac-Vektor umfasst, der ein grün fluoreszierendes Protein (GFP) codiert, zur Selektion von transgenem Bombyx mori.

3. Transgene Seidenraupe, hergestellt nach den Verfahren von Anspruch 1 oder Anspruch 2, wobei die transgene Seidenraupe ein rekombinantes chimäres Gen aufweist, das die Sequenz codiert, die die Kombination von Spinnenseiden-Elastizitätsmotivsequenzen und den Spinnenseiden-Festigkeitsmotivsequenzen umfasst,
wobei die Kombination (A2S8)x14 ist, so dass A2 8 Kopien eines putativen Flagelliform-Seiden-elastischen Motivs GPGGA angibt und S8 ein putatives Dragline-Seiden-Festigkeitsmotiv GGPSGPGS(A)8 angibt, und
wobei die transgene Seidenraupe in der Lage ist, ein chimäres Seidenraupen- / Spinnenseidenprotein aus dem rekombinanten chimären Gen zu erzeugen, wobei das chimäre Seidenraupen- / Spinnenseidenprotein geeignet ist zur Herstellung einer chimären Spinnenseiden- / Seidenraupenfaser mit einer Zugfestigkeit, die 2x größer ist als eine native Seidenraupenfaser.

4. Herstellungsverfahren zur Produktion von chimären Spinnenseiden-Mischfasern, umfassend:
a.) Herstellen der transgenen Seidenraupe nach Anspruch 3;
b.) es der transgenen Seidenraupe ermöglichen, unter geeigneten physiologischen Bedingungen, die für die Seidenraupe nativ sind, einen Kokon zu produzieren;
c.) Sammeln der Kokons und Gewinnen der chimären Spinnenseidenfaser.

5. Genetisches Konstrukt, das gestaltet ist, um Fibroin-Schwerketten (fhc)-Spinnenseiden-Chimären zu exprimieren, in denen ein synthetisches Spinnenseidenprotein von N- und C-terminalen Fragmenten eines Bombyx mori-fhc-Proteins flankiert ist, wobei das genetische Konstrukt in einer 5' zu 3'-Richtung umfasst:
einen Hauptpromotor von dem B. mori fhc-Gen;
ein Upstream-Enhancer-Element von dem B. mori fhc-Gen;
einen basalen Promotor von dem B. mori fhc-Gen;
eine N-terminale Domäne (NTD) von dem B. mori fhc-Gen;
verschiedene synthetische Spinnenseidenproteinsequenzen, die in-frame positioniert sind, mit einer Translationsinitiationsstelle, die stromaufwärts in der NTD lokalisiert ist, wobei die verschiedenen synthetischen Spinnenseidenproteinsequenzen ein chimäres Spinnenseidenprotein mit 14 wiederholten Untereinheiten umfassen, wobei jede Untereinheit 8 Kopien eines putativen Flagelliform-Seiden-elastischen Motivs GPGGA und eines putativen Dragline-Seiden-Festigkeitsmotivs GGPSGPGS(A)8 aufweist, und
eine fhc C-terminale Domäne (CTD) von dem B. mori fhc-Gen, die Translationsterminations- und RNA-Polyadenylierungsstellen umfasst.

6. Genetisches Konstrukt nach Anspruch 5, wobei die verschiedenen synthetischen Spinnenseidenproteinsequenzen 14 wiederholte Untereinheiten umfassen, wobei jede Untereinheit 8 Kopien des putativen Flagelliform-Seiden-elastischen Motivs GPGGA und des putativen Dragline-Seiden-Festigkeitsmotivs GGPSGPGS(A)8 aufweist, und wobei das genetische Konstrukt ferner eine EGFP-Sequenz stromaufwärts der chimären Spinnenseidenproteinsequenz aufweist.

7. Genetisches Konstrukt nach Anspruch 5, wobei das genetische Konstrukt der piggyBac-Vektor wie in Fig. 15 oder Fig. 16 definiert ist.

## Revendications

1. Procédé de préparation d'un ver à soie *Bombyx mori* transgénique capable d'exprimer une protéine de soie d'araignée chimérique stable appropriée pour un assemblage en une fibre composite de soie d'araignée, ladite protéine de soie d'araignée chimérique étant codée par une séquence comprenant une séquence de motif de flexibilité de soie d'araignée et une séquence de motif de résistance de soie d'araignée, ledit procédé comprenant :
a.) l'insertion d'un vecteur piggyBac tel que défini sur la Figure 15 ou la Figure 16, chacun ayant une cassette génétique recombinante codant pour une combinaison d'une séquence de motif de flexibilité de soie d'araignée et d'une séquence de motif de résistance de soie d'araignée définies par 14 sous-unités répétées, chaque sous-unité ayant 8 copies d'un motif putatif élastique de soie flagelliforme GPGGA et un motif putatif de résistance de soie porteuse GGPSGPGS(A)8, dans un œuf de *Bombyx mori* mutant pour obtenir des œufs de *Bombyx mori* injectés ;
b.) le fait de laisser les œufs éclore dans des conditions d'incubation appropriées pour obtenir des larves ;
c.) le fait de permettre aux larves de vieillir dans des conditions d'incubation appropriées ; et
d.) la sélection d'un ver à soie *Bombyx mori* transgénique.

2. Procédé selon la revendication 1 dans lequel la cassette génétique recombinante comprend le vecteur piggyBac défini sur la Figure 16 codant pour une protéine fluorescente verte (GFP) pour la sélection d'un *Bombyx mori* transgénique.

3. Ver à soie transgénique élaboré d'après les procédés selon la revendication 1 ou la revendication 2, le ver à soie transgénique ayant un gène chimérique recombinant codant pour la séquence comprenant la combinaison de séquences de motif d'élasticité de soie d'araignée et de séquences de motif de résistance de soie d'araignée,
dans lequel la combinaison est (A2S8)xl4 de sorte que A2 indique 8 copies d'un motif putatif élastique de soie flagelliforme GPGGA et S8 indique un motif putatif de résistance de soie porteuse GGPSGPGS(A)8, et
dans lequel le ver à soie transgénique est capable de produire une protéine de ver à soie/soie d'araignée chimérique à partir du gène chimérique recombinant, la protéine de ver à soie/soie d'araignée chimérique appropriée pour la production d'une fibre de soie d'araignée/ver à soie chimérique ayant une résistance à la traction deux fois supérieure à celle d'une fibre de ver à soie native.

4. Procédé de fabrication pour la production d'une fibre composite de soie d'araignée chimérique comprenant :
a.) la préparation du ver à soie transgénique selon la revendication 3 ;
b.) le fait de permettre au ver à soie transgénique de produire un cocon dans des conditions physiologiques appropriées natives du ver à soie ;
c.) la collecte des cocons et l'extraction d'une fibre de soie d'araignée chimérique.

5. Construction génétique conçue pour exprimer des chimères de soie d'araignée à chaîne lourde de fibroïne (fhc) dans lesquelles une protéine de soie d'araignée synthétique est flanquée de fragments N- et C-terminaux d'une protéine de fhc de *Bombyx mori,* la construction génétique comprenant, dans une direction 5' vers 3' :
un promoteur majeur provenant du gène de fhc de *B. mori ;*
un élément stimulateur amont provenant du gène de fhc de *B. mori ;*
un promoteur basal provenant du gène de fhc de *B. mori ;*
un domaine N-terminal (NTD) provenant du gène fhc de *B. mori ;*
diverses séquences de protéines de soie d'araignée synthétique positionnées dans le cadre avec un site d'initiation de traduction situé en amont dans le NTD, dans laquelle les diverses séquences de protéines de soie d'araignée synthétique comprennent une protéine de soie d'araignée chimérique ayant 14 sous-unités répétées, chaque sous-unité ayant 8 copies d'un motif putatif élastique de soie flagelliforme GPGGA et un motif putatif de résistance de soie porteuse GGPSGPGS(A)8 ; et
un domaine C-terminal (CTD) de fhc provenant du gène de fhc de *B. mori* qui comporte des sites de terminaison de traduction et de polyadénylation d'ARN.

6. Construction génétique selon la revendication 5, dans laquelle les diverses séquences de protéines de soie d'araignée comprennent 14 sous-unités répétées, chaque sous-unité ayant 8 copies du motif putatif élastique de soie flagelliforme GPGGA et le motif putatif de résistance de soie porteuse GGPSGPGS(A)8, et dans laquelle la construction génétique comprend en outre une séquence EGFP en amont de ladite séquence de protéine de soie d'araignée chimérique.

7. Construction génétique selon la revendication 5, dans laquelle la construction génétique est le vecteur piggyBac tel que défini sur la Figure 15 ou la Figure 16.
